# FASCICULE DE BREVET EUROPEEN

(11) **EP 1 957 490 B1**
(45) Date de publication et mention de la délivrance du brevet: **06.01.2010**
(21) Numéro de dépôt: 06831144.8
(22) Date de dépôt: 21.11.2006
(51) Int. Cl.: C07D 471/04, A61P 9/00, A61K 31/454

(54) **NOUVEAUX DERIVES D'INDOLIZINE, LEUR PROCEDE DE PREPARATION ET LES COMPOSITIONS THERAPEUTIQUES LES COMPRENANT**
NEUE INDOLIZINDERIVATE, VERFAHREN ZU DEREN HERSTELLUNG UND THERAPEUTISCHE ZUSAMMENSETZUNGEN, DIE DIESE ENTHALTEN
NOVEL INDOLIZINE DERIVATIVES, METHOD FOR PREPARING SAME AND THERAPEUTIC COMPOSITIONS COMPRISING SAME

(30) Priorité: 23.11.2005 FR 0511849
(43) Date de publication de la demande: 20.08.2008
(73) Titulaire: sanofi-aventis, 75013 Paris (FR)
(72) Inventeur: GAUTIER, Patrick, F-92160 Antony (FR); MARCHIONNI, David, F-92160 Antony (FR); ROCCON, Alain, F-92160 Antony (FR); TONNERRE, Bernard, F-92160 Antony (FR); WAGNON, Jean, F-92160 Antony (FR)
(74) Mandataire: Romanowski, Caroline
(86) Numéro de dépôt international: PCT/FR2006/002551
(87) Numéro de publication internationale: WO 2007/060318

(56) Documents cités:
- EP-A- 0 471 609
- FR-A- 2 341 578

## Description

La présente invention a pour objet de nouveaux dérivés d'indolizine, leur procédé de préparation et lés compositions pharmaceutiques les contenant.

Dans FR 2 341 578 et EP 471 609, il est décrit des dérivés d'indolizine qui possèdent de remarquables propriétés pharmacologiques notamment des propriétés anti-arythmiques puisque ces dérivés se sont révélés capables de supprimer ou de prévenir les troubles du rythme ventriculaire et auriculaire.
La plupart des composés décrits ont des propriétés électrophysiologiques des classes 1, 2, 3 et 4 de la classification de Vaughan-Williams qui confèrent en plus de leurs propriétés antiarythmiques, des propriétés bradycardisantes, anti-hypertensives et anti-adrénergiques a et β non compétitives. Ces propriétés rendent les composés en question très utiles dans le traitement de certains syndromes pathologiques du système cardio-vasculaire en particulier dans le traitement de l'angine de poitrine, de l'hypertension, de l'arythmie ventriculaire ou supraventriculaire. De même, ces composés sont utilisés dans le traitement de l'insuffisance cardiaque, de l'infarctus du myocarde compliqué ou non d'insuffisance cardiaque ou pour la prévention de la mortalité post-infarctus.
Néanmoins, ces composés présentent l'inconvénient de n'être pas ou très peu solubles dans l'eau, en particulier à pH≥ 4 et surtout à pH physiologique.

L'amiodarone, qui est un antiarythmique auriculaire et ventriculaire actif par voies orale et intraveineuse, est une molécule insoluble dans l'eau; la solution injectable contient donc des solvants comme le polysorbate 80 et l'alcool benzylique. Ces solvants induisent chez le patient des effets hypotenseur et inotrope négatif. La solution injectable provoque aussi des intolérances veineuses locales que l'on évite en préconisant une injection centrale en milieu hospitalier spécialisé.

La dronedarone, dérivé benzofurane, ne contenant pas d'iode dans sa structure chimique contrairement à l'amiodarone, est également un antiarythmique auriculaire et ventriculaire actif par voie orale et intraveineuse.
Son faible niveau de solubilité (solubilité dans l'eau de S=0,247 mg/ml à pH=3) limite notablement la possibilité d'être préparé et stocké sous forme de produit injectable.
Ce faible niveau de solubilité limite notablement leurs possibilités d'être préparés et stockés sous forme de produit injectable.

On a maintenant découvert, dans le cadre de l'invention, de nouveaux dérivés de l'indolizine qui présentent une bonne solubilité dans l'eau, tout en gardant, voire en améliorant, leur propriétés pharmacologiques notamment leurs propriétés antiarythmiques. Leur bonne solubilité dans l'eau en particulier à pH≥ 4 permet notamment de réaliser des formes pharmaceutiques injectables.
Les composés de la présente invention sont des molécules, solubles dans l'eau, que l'on peut administrer par voie intraveineuse dans une solution physiologique (chlorure de sodium à 0,9%) ou glucosée, et qui possèdent les propriétés électrophysiologiques, hémodynamiques et antiarythmique de l'amiodarone.
Par ailleurs, ces nouveaux composés offrent aussi une bonne stabilité métabolique.
L'invention a donc pour but de proposer des dérivés d'indolizine comportant une chaîne aminoalkylbenzoyle représentés par la formule (I) ci-dessous.

La présente invention concerne ainsi de nouveaux dérivés d'indolizine de formule générale (I) :
dans laquelle, 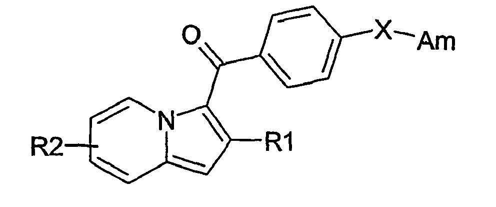
X représente un radical -(CH₂)n- avec n étant un nombre entier allant de 1 à 6,
R₁ représente un radical alkyle en C₁₋C₈ linéaire, ramifié ou cyclique ;
Am représente un groupe NR₃R₄ dans lequel R₃ et R₄ sont identiques ou différents et représentent indépendamment l'un de l'autre
   - un atome d'hydrogène,
   - un radical alkyle en C₁-C₈ linéaire, ramifié ou cyclique, ou en C₁-C₃ substitué par un radical cycloalkyle en C₃-C₆,
   - ou un radical (CH₂)I-O-B, dans lequel B représente un atome d'hydrogène
   ou un radical alkyle (CH₂)k, avec I et k étant des nombres entiers I ≥ 2 et I+k ≤ 6
   ou R₃ et R₄ forment ensemble avec l'atome d'azote auquel ils sont attachés un hétérocycle de 4 à 6 chaînons pouvant éventuellement contenir un ou plusieurs hétéroatomes choisis parmi N, O et portant éventuellement un ou plusieurs substituants choisis parmi un radical alkyle en C₁-C₆ linéaire, ramifié ou cyclique,
R₂ représente un atome d'hydrogène, un radical alkyle en C₁-C₆ linéaire, ramifié ou cyclique,
et leurs sels pharmaceutiquement acceptables.

L'invention concerne plus particulièrement des dérivés d'indolizine tels que définis plus haut caractérisés en ce que X représente un radical (CH₂)n- avec n étant un nombre entier allant de 3 à 4.

Plus particulièrement, les dérivés d'indolizine selon l'invention sont caractérisés en ce que R₁ représente un radical alkyle en C₁-C₄.

Plus particulièrement, les dérivés d'indolizine selon l'invention sont caractérisés en ce que R₃ et R₄ représentent indépendamment l'un de l'autre un radical alkyle en C₁-C₄ linéaire.

En particulier, les dérivés d'indolizine selon l'invention sont caractérisés en ce que R₃ et R₄ forment ensemble un groupement pipéridinyle ou pipérazinyle portant éventuellement un ou plusieurs radical méthyle.

En particulier, les dérivés d'indolizine selon l'invention sont caractérisés en ce que R₂ représente un atome d'hydrogène.

Les composés de formule (I) peuvent exister à l'état de bases ou salifiés par des acides ou des bases, notamment des acides ou des bases pharmaceutiquement acceptables. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I), font également partie de l'invention.

Les composés de formule (I) peuvent également exister sous forme d'hydrates ou de solvants, à savoir sous forme d'associations ou de combinaisons avec une ou plusieurs molécules d'eau ou avec un solvant. De tels hydrates et solvats font également partie de l'invention.

L'invention se rapporte également aux sels pharmaceutiquement acceptables des composés de formule (I) formés à partir d'un acide organique ou inorganique.
Comme exemples de sels organiques de ce genre, on peut citer les malonate, dodécanoate, oxalate, maléate, fumarate, méthanesulfonate, benzoate, ascorbate, palmoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théo-phylline acétate ainsi que les sels formés à partir d'un acide aminé tels que le sel de lysine ou d'histidine.
Comme sels inorganiques de ce genre, on peut citer les chlorhydrate, bromhydrate, sulfate, sulfamate, phosphate et nitrate.
Plus particulièrement, l'invention concerne des dérivés d'indolizine tels que définis plus haut caractérisés en ce que le sel pharmaceutiquement acceptable est choisi parmi l'oxalate et le chlorhydrate.

L'invention a aussi pour objet la préparation des composés de formule (I).
La présente invention se rapporte ainsi au procédé de préparation des dérivés de formule (I) tels que définis plus haut comprenant la réaction d'une amine H-Am sur un composé représenté par la formule (II) : 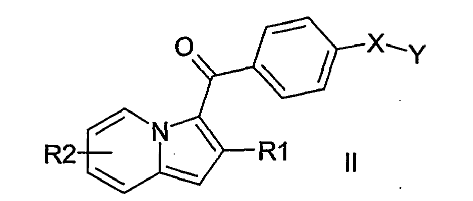 dans laquelle Y représente un atome d'halogène et les autres substituants sont tels que définis précédemment.

L'invention se rapporte aussi à un procédé comprenant un procédé de préparation du composé de formule (II) telle que définie plus haut et comprenant la réaction d'un composé représenté par la formule (III) : 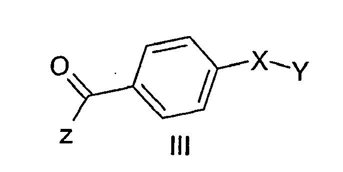 sur un composé représenté par la formule (IV):

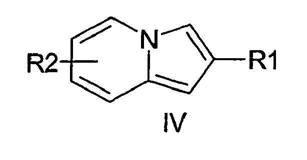

Ainsi, selon l'invention, les composés de formule (I) dans laquelle X représente un radical -(CH₂)n- tel que défini précédemment sont préparés selon le schéma de synthèses suivant : 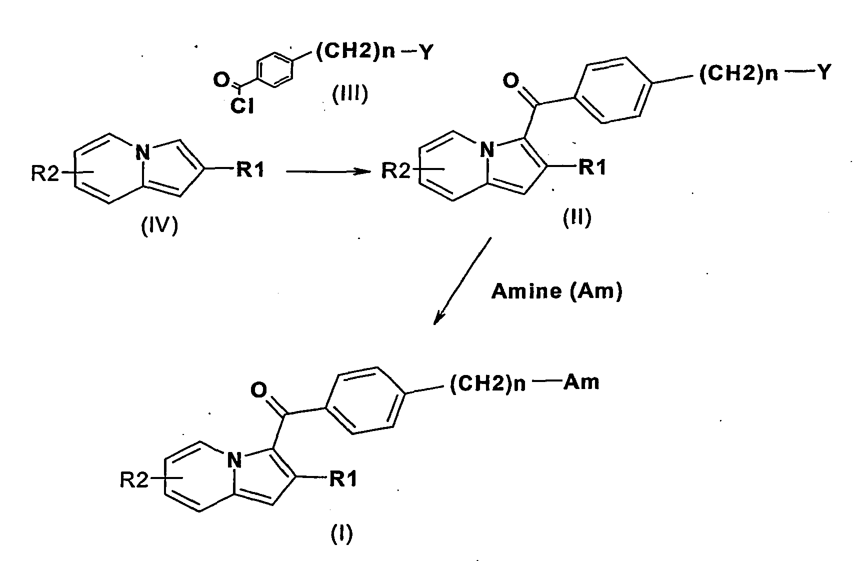 dans lequel Y représente un halogène par exemple Cl ou Br et n, les groupes R₁, R₂, Am ont les mêmes significations que citées précédemment.

Pour la préparation des composés de formule (III) on pourra se référer à US 6949583.
Pour la préparation des composés de formule (IV) on pourra se référer à la publication EUR J. MED. CHEM-CHEMICAL THERAPEUTICA, NOV.-DEC. 1975-10, N°6, p. 579 à 584 et au brevet de LABAZ US 4103012.
De même pour la préparation des dérivés d'indolizine de formule (I) qui s'effectue par condensation d'une amine secondaire de formule H-Am sur le dérivé chloré de formule (II) et pour la préparation des composés de formule (II) qui s'effectue par la réaction des composés de formule (III) sur les dérivés de formule (IV), on pourra se référer au document US4103012.

Selon l'invention, les composés de formule (I) lorsque X représente un groupement de formule CH₂-Z-(CH₂)₂-, dans laquelle Z représente un groupe O(CH₂)m- tel que défini précédemment, on pourra se référer à US 6949583 et à Monatsh.chem.;129;3;1998;309-318., ainsi qu'au schéma de synthèse suivant : 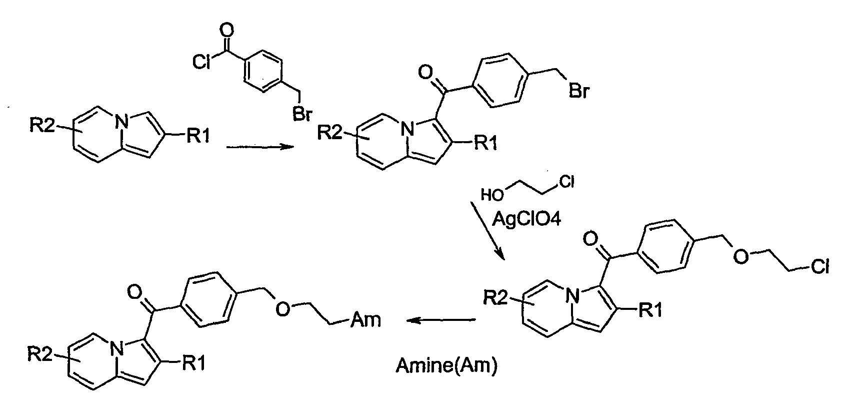 dans lequel les groupes R₁, R₂, Am ont les mêmes significations que citées précédemment.

L'invention a aussi également pour objet une composition pharmaceutique ou vétérinaire, caractérisée en ce qu'elle contient comme principe actif au moins un dérivé d'indolizine de formule (I) tel que défini plus haut, ou un sel pharmaceutiquement acceptable de ce dernier, en association avec un véhicule pharmaceutique ou un excipient approprié.
Par exemple les compositions pharmaceutiques de la présente invention peuvent être formulées pour l'administration orale, sublinguale, sous-cutanée, intramusculaire, intraveineuse, transdermique ou rectale. De préférence, les compositions sont présentées sous forme injectable compte tenu des bonnes caractéristiques de solubilité des dérivés de l'invention.
Pour ce qui concerne l'unité d'administration, celle-ci peut prendre la forme, par exemple, d'un comprimé, d'une dragée, d'une capsule, d'une gélule, d'une poudre, d'une suspension, d'un sirop ou encore de granules pour l'administration orale, d'un suppositoire pour l'administration rectale ou d'une solution ou suspension pour l'administration parentérale.
Les compositions pharmaceutiques de l'invention pourront comprendre, par unité d'administration, par exemple de 50 à 500. mg en poids d'ingrédient actif pour l'administration orale, de 50 à 200 mg d'ingrédient actif pour l'administration rectale et de 50 à 150 mg d'ingrédient actif pour l'administration parentérale.
Suivant la voie d'administration choisie, les compositions pharmaceutiques ou vétérinaires de l'invention seront préparées en associant au moins un des composés de formule (I) ou un sel pharmaceutiquement acceptable de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.
Lorsqu'il s'agit de comprimés, ceux-ci peuvent être traités de telle sorte qu'ils présentent une activité prolongée ou retardée et qu'ils libèrent d'une façon continue une quantité prédéterminée de principe actif.

L'invention concerne également des dérivés d'indolizine de formule générale (I) tels que définis plus haut pour leur utilisation en tant que médicament. La présente invention concerne ainsi des médicaments comprenant les dérivés d'indolizine de formule générale (I) tels que définis plus haut.

Les dérivés d'indolizine de formule générale (I) de la présente invention tels que définis plus haut peuvent être utiles pour la préparation d'un médicament destiné au traitement des syndromes pathologiques du système cardio-vasculaire. Les dérivés d'indolizine de formule générale (I) de la présente invention tels que définis plus haut peuvent être utiles plus particulièrement pour la préparation d'un médicament destiné au traitement de l'angine de poitrine, de l'hypertension, de l'arythmie ventriculaire, de l'arythmie supraventriculaire, en particulier de la fibrillation auriculaire, de l'insuffisance cardiaque, de l'infarctus du myocarde compliqué ou non d'insuffisance cardiaque ou pour la prévention de la mortalité pose-infarctus

Les résultats de tests pharmacologiques effectués en vue de déterminer des propriétés des composés de l'invention sur le système cardiovasculaire sont répertoriés ci-dessous.

### I. Arythmies ventriculaires

Le but de ce test est de déterminer la capacité des composés de l'invention à assurer une protection contre les arythmies provoquées par reperfusion d'une artère coronaire préalablement ischémiée. A cet effet, on a utilisé la méthode rapportée par MANNING A.S. et coll. dans Circ. Res. 1984, 55 : 545-548 modifiée comme suit :
On anesthésie d'abord des rats, répartis en lots, avec du pentobarbital sodique (60mg/kg par voie intrapéritonéale) puis on les intube afin de les maintenir sous respiration assistée.
On leur place ensuite une canule dans une veine jugulaire pour administration intraveineuse, un cathéter dans une artère carotide pour mesure de la pression artérielle, ainsi que des électrodes cutanées pour mesure de l'ECG. Après équilibration des paramètres, on administre une dose intraveineuse du composé à étudier et 5 minutes plus tard, après réalisation d'une thoracotomie, on place une boucle de ligature autour de l'artère coronaire descendante antérieure gauche, à proximité immédiate de son origine. On provoque alors l'occlusion de cette artère pendant 5 minutes par traction sur les extrémités de la ligature. La reperfusion est obtenue par relâchement de la tension.
On évalue alors les arythmies induites par cette reperfusion.
Un test analogue a été pratiqué après administration orale du composé Dans ce cas, le composé à étudier est administré de 60 à 120 minutes avant la ligature de l'artère coronaire descendante antérieure gauche.
Les résultats de ces tests ont montré que les composés de l'invention, administrés à des doses comprises entre 0,3 et 10 mg/kg par voie intraveineuses et 30 à 100 mg/kg par voie orale protègent de manière significative, voire totale, des arythmies ventriculaires les animaux traités.

### II. Propriétés antiadrénergiques

Le but de ce test est de déterminer la capacité des composés de l'invention à réduire l'augmentation de la pression sanguine induite par la phényléphrine (effet anti-α) et l'accélération de la fréquence cardiaque induite par l'isoprénaline (effet anti-β) chez le chien préalablement anesthésié au pentobarbital et chloralose.
On détermine d'abord pour chaque chien la dose de phényléphrine (5 µg/kg) qui provoque une augmentation de la pression artérielle comprise entre 25 et 40 mm Hg et la dose d'isoprénaline (0,5 ou 1 µg/kg) qui devra entraîner une augmentation de la fréquence cardiaque comprise entre 40 et 110 battements/minute.
On injecte alternativement toutes les 10 minutes les doses de phényléphrine et d'isoprénaline ainsi déterminées et après obtention de 2 réponses de référence successives, on administre une dose du composé à étudier par voie intraveineuse .

### - Effet anti-α

On enregistre le pourcentage de réduction, par le composé de l'invention, de l'hypertension provoquée comparativement à l'hypertension de référence obtenue avant injection de ce composé.

### - Effet anti-β

On enregistre le pourcentage de réduction, par le composé à étudier, de l'accélération provoquée de la fréquence cardiaque.

Les résultats de ces tests montrent qu'aux doses de 3 et 10 mg/kg, les composés de l'invention présentent des effets anti-α et/ou anti-β se traduisant par des réductions de l'hypertension provoquée et/ou de l'augmentation provoquée de la fréquence cardiaque, allant jusqu'à 50%.

### III. Arythmies auriculaires

Le but de ce test est d'évaluer l'efficacité des composés de l'invention vis-à-vis de l'arythmie auriculaire (vulnérabilité auriculaire gauche) induite par des extra-stimulus chez le porc anesthésié selon la méthode décrite dans Naunyn-Schmiedeberg's Arch Pharmacol 2001 ; 363 : 166-174.

Les composés à étudier sont administrés à la dose de 3 mg/kg en perfusions intraveineuses lentes de 5 minutes. Les paramètres hémodynamiques et électrocardiographiques ainsi que les périodes réfractaires auriculaires droite et gauche sont déterminés avant et après l'administration du composé à étudier.

A la dose de 3 mg/kg les composés de l'invention abolissent généralement 100% des épisodes non soutenus de fibrillation ou flutter auriculaire induits par des extra-stimulus précoces. A cette dose, on observe des augmentations significatives des périodes réfractaires effectives auriculaires pour différentes valeurs basales de la période cardiaque.

### IV. Effets des canaux ioniques cardiaques

Le but de ce test est de rechercher les effets des composés de l'invention sur les canaux ioniques cardiaques.

Les composés de l'invention (1 à 5 µM) inhibent les perméabilités des canaux de gènes humains hERG (IKᵣ) et hKv1.5 (IKᵤᵣ) exprimés dans la lignée cellulaire CHO (Chinese hamster ovary). Ils inhibent aussi les courants natifs des canaux sodique (INa), calcique (ICa_{L}) et potassiques activés par l'acétylcholine (IK_{(ACh)}) et ATP-dépendant (IK_{ATP}) des cardiomyocytes de cobaye, et des canaux potassiques (Ito et Isus) des cardiomyocytes de rat.

Les composés de l'invention sont donc des inhibiteurs de multiples canaux ioniques cardiaques.

### V. Toxicité

La toxicité des composés de l'invention s'est révélée compatible avec leur utilisation en thérapeutique.

### VI. Solubilité.

Les tests de solubilité des composés de l'invention sont réalisés à pH≥ 4 (à partir d'un tampon phosphate pH=6 0,1 M) par HPLC (chromatographie liquide haute performance) en utilisant un gradient H₂O / CH₃CN / CH₃SO₃H par rapport à un échantillon de référence (une solution diluée du produit à tester qui sert d'étalon interne).
Les résultats de solubilité S et de pH sont présentés dans le tableau suivant et sont exprimés en mg/ml.

Généralement les composés de formule (I) de la présente invention présentent une solubilité supérieure ou égale à 1 mg/ml à pH≥4, certains d'entre eux pouvant avoir une solubilité supérieure à 8 mg/ml voire à 10 mg/mL

### TABLEAU DES EXEMPLES

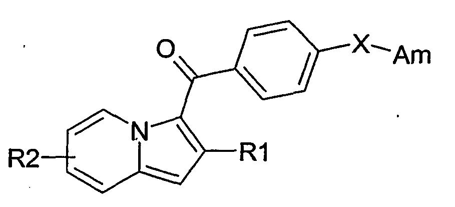

Avec X= (CH2)n

| **composé n°** | **sel** | **n** | **R1** | **R2** | **Am** | **S/pH** |
|---|---|---|---|---|---|---|
| 1 | chlorhydrate | 3 | C4H9 | H | 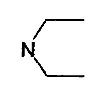 | 8,81/5,4 |
| 2 | oxalate | 3 | C2H5 | H | | 7,47/4,07 |
| 3 | chlorhydrate | 3 | nC3H7 | H | | > 10/5,82 |
| 4 | oxalate | 4 | C4H9 | H | | >10/4,10 |
| 5 | oxalate | 4 | C2H5 | H | | >10/3, 96 |
| 6 | chlorhydrate | 4 | nC3H7 | H | | >10/5,79 |
| 7 | chlorhydrate | 3 | C4H9 | H | 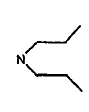 | 8,57/5,65 |
| 8 | chlorhydrate | 3 | C2H5 | H | | 8,6/5,82 |
| 9 | chlorhydrate | 3 | C3H7 | H | | 5,29/5,82 |
| 10 | chlorhydrate | 4 | C4H9 | H | | >10/5,81 |
| 11 | chlorhydrate | 4 | C2H5 | H | | >10/5,82 |
| 12 | chlorhydrate | 3 | ipr | H | | 1,2/4,3 |
| 13 | chlorhydrate | 3 | CH3 | H | | 1,61/7,2 |
| 14 | oxalate | 4 | ipr | H | | 2,33/4,10 |
| 15 | chlorhydrate | 4 | CH3 | H | | 1,56/6,33 |
| 16 | chlorhydrate | 4 | C3H7 | H | | >10/5,81 |
| 17 | chlorhydrate | 3 | C4H9 | H | 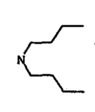 | >10/4,88 |
| 18 | chlorhydrate | 3 | C2H5 | H | | 6,51/5,42 |
| 19 | chlorhydrate | 3 | C3H7 | H | | 4,07/5,27 |
| 20 | oxalate | 4 | C4H9 | H | | 2,95/4,02 |
| 21 | oxalate | 4 | C2H5 | H | | 6,9/4,08 |
| 22 | oxalate | 4 | C3H7 | H | | 4,08/4,58 |
| 23 | chlorhydrate | 3 | C4H9 | H | 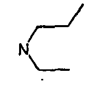 | >10/6,06 |
| 24 | oxalate | 3 | C2H5 | H | | 5,92/4,51 |
| 25 | chlorhydrate | 3 | ipr | H | | 1,71/6,4 |
| 26 | chlorhydrate | 3 | CH3 | H | | 1,49/7,14 |
| 27 | oxalate | 4 | ipr | H | 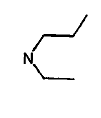 | 2,17/6,5 |
| 28 | chlorhydrate | 4 | CH3 | H | | 1,54/7,01 |
| 29 | chlorhydrate | 3 | C3H7 | H | | >10/5,94 |
| 30 | chlorhydrate | 4 | C4H9 | H | | >10/5,85 |
| 31 | oxalate | 4 | C2H5 | H | | >10/4,05 |
| 32 | chlorhydrate | 4 | C3H7 | H | | 5,64/5,60 |
| 33 | oxalate | 3 | C4H9 | H | 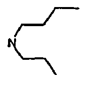 | 3,88/5,36 |
| 34 | oxalate | 3 | C4H9 | H | 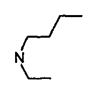 | 5,02/4,14 |
| 35 | oxalate | 3 | C4H9 | H | 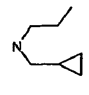 | 4,4/4,75 |
| 36 | chlorhydrate | 4 | C3H7 | H | 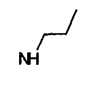 | 2,24/6,3 |
| 37 | chlorhydrate | 3 | C4H9 | H | | 1,78/7,14 |
| 38 | chlorhydrate | 3 | C4H9 | H | 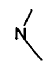 | 1,42/6,7 |
| 39 | chlorhydrate | 3 | C4H9 | H | 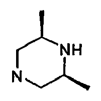 | >10/4,22 |
| 40 | chlorhydrate | 3 | C4H9 | H | 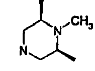 | 9,93/4,3 |
| 41 | oxalate | 3 | C4H9 | H | 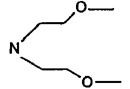 | 7,19/3,95 |
| 42 | oxalate | 3 | C4H9 | H | 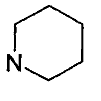 | 1,66/5,18 |
| | | | | | | |

| **composé n°** | **sel** | **n** | **R1** | **R2** En position 6 | **Am** | **S/pH** |
|---|---|---|---|---|---|---|
| 43 | chlorhydrate | 3 | C4H9 | CH3 | 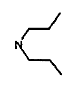 | 4,46/5,65 |
| 44 | chlorhydrate | 3 | C4H9 | C2H5 | | >10/6,05 |
| 45 | chlorhydrate | 3 | C4H9 | CH3 | 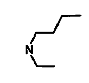 | 5,8/5,95 |
| 46 | chlorhydrate | 3 | C4H9 | C2H5 | | 8,92/5,92 |

Les Exemples, non limitatifs suivants, illustrent la préparation des composés et compositions de l'invention :
Les spectres de résonance magnétique du proton (RMN ¹H), tels que décrits ci-dessous, sont enregistrés à 200 MHz dans du DMSO-d₆, en utilisant le pic du DMSO-d₆ comme référence.
Les déplacements chimiques δ sont exprimés en parties par million (ppm). Les signaux observés sont exprimés ainsi : s : singulet; se : singulet élargi ; d : doublet ; d.d : doublet dédoublé ; t : triplet ; td : triplet dédoublé ; q : quadruplet ;
qui : quintuplet ; m : massif ; mt : multiplet ; sxt : sextuplet.

### Exemple 1 Chlorhydrate de (2-butyl-6-éthylindolizin-3-yl){4-[3-(dipropylamino)propyl]phényl}méthanone (composé 44)

### a) 1-bromohexane-2-one

A une solution de 58,6 g (0,976 mole) d'urée dans 210 ml d'acide acétique, on ajoute lentement à la température ambiante 60 g (0,599 mole) de 2-hexanone. On refroidit le milieu à 0° C par un bain de glace, puis on ajoute goutte à goutte 33 ml de brome (0,640 mole) et on agite 18 heures à la température ambiante. On verse sur de l'eau (1500 ml), on extrait au chlorure de méthylène, on lave la phase organique avec une solution d'hydrogénocarbonate de sodium, puis on sèche sur sulfate de sodium et on concentre la solution.
Le résidu est distillé sous pression réduite. On récupère ainsi 76 g du composé attendu (point d'ébullition : 80-90 °C /18mm de Hg) contaminé par un peu de son isomère, le 3-bromohexanone (6 %).

### b) Bromure de N-(hexanone-2-yl)méthyl-2-éthyl-5-pyridinium

On ajoute lentement 21,6 g (0,178 mole) de 5-éthyl-2-méthylpyridine à 40 g de bromo-1-hexanone-2 (0,223 mole) et on agite 3 heures à la température ambiante. On obtient un milieu visqueux que l'on utilise tel quel pour l'étape suivante.

### c) 2-butyl-6-éthyl-indolizine

A une solution du produit décrit précédemment dans 300 ml d'éthanol, on ajoute goutte à goutte une solution de 65 g de triéthylamine dans 370 ml d'éthanol, puis on porte au reflux 18 heures. On concentre sous vide, on reprend par de l'acétate d'éthyle, on lave à l'eau, puis on sèche la phase organique sur sulfate de sodium et on concentre à sec. On purifie alors par flash chromatographie sur silice (éluant : heptane/Chlorure de méthylène 50/50). De cette manière, on obtient 15 g du composé désiré sous la forme d'une huile jaune.
Rendement : 34 %.

### d) (2-butyl-6-éthylindolizin-3-yl)[4-(3-chloropropyl)phényl]méthanone

On chauffe à 50 °C pendant 18 heures un mélange de 5 g (27 mmoles) du composé obtenu à l'étape précédente et de 5,9 g (27 mmoles) de chlorure de 4-(3-chloropropyl)benzoyle. On reprend par de l'eau, on extrait au dichlorométhane, on lave la phase organique avec une solution de carbonate de sodium, puis on sèche sur sulfate de sodium et on concentre à sec. On purifie alors par flash chromatographie sur alumine (éluant : heptane/chlorure de méthylène 50/50). De cette manière, on obtient 8 g du composé désiré sous la forme d'une huile.
Rendement : 76 %

### e) (2-butyl-6-éthylindolizin-3-yl){4-[(3-(dipropylamino)propyl]phényl}méthanone

Dans un réacteur, on dissout 4 g (10,4 mmoles) du composé obtenu à l'étape précédente dans 50 ml de méthylisobutyl-cétone et on ajoute à ce milieu réactionnel, 2,7 g (27 mmoles) de dipropylamine, 0,31 g (2 mmoles) d'iodure de sodium et on chauffe à 110 °C pendant 48 heures. On verse dans l'eau, on extrait au dichlorométhane, on lave avec une solution d'hydrogénocarbonate de sodium, on sèche sur sulfate de sodium et concentre à sec. On purifie alors par flash chromatographie sur alumine (éluant : Chlorure de méthylène-acétate d'éthyle 80/20). De cette manière, on obtient 3,9 g du composé désiré.
Rendement : 84 %

### f) Chlorhydrate de (2-butyl-6-éthylindolizin-3-yl){4-[3-(dipropylamino)propyl]phényl}méthanone

A une solution du produit décrit précédemment (3,9 g) dans l'éther (400 ml), on ajoute 6,5 ml d'une solution d'éther chlorhydrique (2 M dans l'éther), on agite 30 minutes et on filtre le sel qui précipite. De cette manière, on obtient 3,7 g du composé désiré.
Rendement: 88 % Fc = 140,7 °C
Spectre de R.M.N. ¹H (200 MHz, (DMSO), δ en ppm) ;
0,65 (t : 3H) ; 0,79 -1,10 (m : 8H) ; 1,11 - 1,44 (m : 5H) ; 1,66 (mt : 4H) ; 2,03 (mt : 2H) ; 2,17 (t : 2H) ; 2,62 (q : 2H) ; 2,74. (t : 2H) ; 2,87 - 3,16 (m : 6H) ; 6,45 (s : 1H) ; 7,15 (d : 1H) ; 7,29 - 7,65 (m : 5H) ; 9,36 (s : 1H) ; 10,49 (se : 1H)

Suivant le procédé décrit ci-dessus dans l'exemple 1 mais en utilisant les produits de départ appropriés on a préparé les composés ci après :
- Chlorhydrate de (2-butyl-6-méthylindolizin-3-yl){4-[3-(dipropylamino)propyl]phényl}méthanone
   Fc=140°C
   Spectre de R.M.N. ¹H (200 MHz, (DMSO), δ en ppm) ; 0,65 (t : 3H) ; 0,79 - 1,10 (m : 8H) ; 1,32 (qui :2H) ; 1,66 (m : 4H) ; 2,03 (mt : 2H) ; 2,17 (t : 2H) ; 2,30 (s : 3H) ; 2,74 (t : 2H) ; 2,87 - 3,16 (m : 6H) ; 6,45 (s : 1H); 7,09 (d : 1H) ; 7,31-7,61 (m : 5H) ; 9,34 (s : 1H) ; 10,60 (se : 1H)
- Chlorhydrate de (2-butyl-6-méthylindolizin-3-yl)(4-{3-[éthyl(propyl)amino]propyl}phényl)méthanone
   Fc=132°C
   Spectre de R.M.N. ¹H (200 MHz, (DMSO), δ en ppm) ; 0,64 (t : 3H) ; 0,79 - 1,09 (m : 5H) ; 1,09 - 1,43 (m : 5H) ; 1,65 (mt : 2H) ; 2,01 (mt : 2H) ; 2,15 (t : 2H) ; 2,29 (s : 3H) ; 2,74 (t : 2H) ; 2,85 - 3,21 (m : 6H) ; 6,44 (s : 1H) ; 7,08 (d : 1H) ; 7,25 - 7,65 (m : 5H) ; 9,33 (s 1H) ; 10,57 (se : 1H)
- Chlorhydrate de (2-butyl-6-éthylindolizin-3-yl)(4-{3-[éthyl(propyl)amino]propyl}phényl)méthanone
   Fc=148,7°C
   Spectre de R.M.N. ¹H (200 MHz, (DMSO), δ en ppm) ; 0,64 (t : 3H) ; 0,79 - 1,10 (m : 5H) ; 1,10 - 1,44 (m : 8H) ; 1,65 (mt : 2H) ; 2,01 (mt : 2H) ; 2,17 (t : 2H) ; 2,62 (q : 2H) ; 2,75 (t : 2H) ; 2,85 - 3,23 (m : 6H) ; 6,45 (s : 1H) ; 7,15 (d : 1H) ; 7,29 - 7,65 (m : 5H) ; 9,36 (s : 1H) ; 10,53 (se : 1H)

### Exemple 2 Oxalate de (2-éthylindolizin-3-yl){4-[3-(diéthylamino)propyl]phényl}méthanone_(composé 2)

### a) (2-éthylindolizin-3-yl)[4-(3-chloropropyl)phényl]méthanone

On chauffe à 50 °C pendant 18 heures un mélange de 8 g (55 mmoles) de 2-éthyl-indolizine et de 13,1 g (60 mmoles) de chlorure de 4-(3-chloropropyl)benzoyle. On reprend par de l'eau, on extrait au dichlorométhane, on lave avec une solution de carbonate de sodium, on sèche la phase organique sur sulfate de sodium et on concentre à sec. On purifie alors par flash chromatographie sur alumine (éluant : cyclohexane/chlorure de méthylène 50/50). De cette manière, on obtient 17 g du composé désiré sous la forme d'une huile. Rendement : 94 %.

### b) (2-éthylindolizin-3-yl){4-[3-(diéthlamino)propyl]phényl}méthanone

Dans un réacteur, on dissout 4 g (12 mmoles) du composé obtenu à l'étape précédente dans 50 ml de méthyl isobutylcétone et on ajoute à ce milieu réactionnel 2,3 g (31 mmoles) de diéthylamine, 0,36 g (2,4 mmoles) d'iodure de sodium, on chauffe à 110°C pendant 48 heures. On verse dans l'eau, on extrait au dichlorométhane, on lave avec une solution d'hydrogénocarbonate de sodium, on sèche sur sulfate de sodium et on concentre à sec. On purifie alors par flash chromatographie sur silice (éluant : méthanol-chorure de méthylène 10/90). De cette manière, on obtient 2,9 g du composé désiré.
Rendement : 65 %

### c) oxalate de (2-éthylindolizin-3-yl){4-[3-(diéthylamino)propyl]phényl}méthanone

On dissout 2,9 g du produit décrit ci-dessus dans 40 ml d'acétate d'éthyle et on ajoute 0,719 g (1 équivalent) d'acide oxalique. On filtre alors le sel qui cristallise. De cette manière, on obtient 2,9 g du composé désiré.
Rendement: 80 % PF = 160 °C
Spectre de R.M.N. ¹H (200 MHz, (DMSO), δ en ppm) ; 0,98 (t : 3H) ; 1,15 (t : 6H) ; 1,96 (m : 2H) ; 2,20 (q : 2H) ; 2,74 (t : 2H) ; 2,90 - 3,19 (m : 6H) ; 6,54 (s : 1H) ; 6,93 (t : 1H) ; 7,21 (t : 1H) ; 7,31 - 7,58 (système AA'-BB' : 4H) ; 7,64 (d : 1H) ; 9,47 (d : 1H) ; 8,39 (se : 2H)

### Exemple 3 Chlorhydrate de(2-butylindolizin-3-yl){4-[3-(dipropylamino)propyl]phényl}méthanone (composé 7)

### a) Bromure de 2-méthyl-1-(2-oxo-hexyl)-pyridinium

On ajoute lentement 30,21 g de 2-picoline (0,32 mole) à 83 g de bromo-1-hexanone-2 (0,46 mole) et agite 4 heures à la température ambiante. On obtient un milieu visqueux que l'on utilise tel quel pour l'étape suivante.

### b) 2-butylindolizine

On dissout du bromure de N-(hexanone-2-yl)méthyl-pyridinium obtenu précédemment dans 100 ml d'éthanol et on ajoute 84 g d'hydrogénocarbonate de sodium en solution dans 630 ml d'eau. On chauffe le milieu réactionnel pendant 3 h à 110 °C. On concentre sous vide, on reprend par de l'eau, on extrait au dichlorométhane, on lave la phase organique avec une solution de chlorure de sodium, on sèche sur sulfate de sodium et on évapore à sec. On purifie le produit obtenu sur colonne de silice et sous pression (éludant: Chlorure de méthylène/heptane 20/80). De cette manière, on obtient 33 g de butyl-2-indolizine.
Rendement : 30 %

### c 2-butylindolizin-3-yl)[4-(3-chloropropyl)phényl]méthanone

On chauffe à 50°C pendant 18 heures, un mélange de 9 g (56 mmoles) de 2-butyl-indolizine et de 12,4 g (57 mmoles) de chlorure de 4-(3-chloropropyl)benzoyle. On reprend par de l'eau, on extrait au dichlorométhane, on lave avec une solution de carbonate de sodium, on sèche la phase organique sur sulfate de sodium et on concentre à sec. On purifie alors par flash chromatographie sur silice (éluant : Chlorure de méthylène- heptane 80/20). De cette manière, on obtient 14 g du composé désiré sous la forme d'une huile.
Rendement : 77 %

### d) (2-butylindolizin-3-yl){4-[3-(dipropylamino)propyl]phényl}méthanone

Dans un réacteur, on dissout 6 g (17 mmoles) du composé obtenu à l'étape précédente dans 60 ml de méthylisobutylcétone et on ajoute à ce milieu réactionnel 4,4 g (44 mmoles) de dipropylamine, 0,5 g (3,4 mmoles) d'iodure de sodium et on chauffe à 110 °C pendant 48 heures. On verse dans l'eau, on extrait au dichlorométhane, on lave avec une solution d'hydrogénocarbonate de sodium, on sèche sur sulfate de sodium et on concentre à sec.
On purifie alors par flash chromatographie sur alumine (éluant : Chlorure de méthylène /méthanol 98/2).
De cette manière, on obtient 6,2 g du composé désiré.
Rendement : 88 %

### e) Chlorhydrate de (2-butylindolizin-3-yl){4-[3-(dipropylamino)propyl]phényl}méthanone

A une solution du produit décrit précédemment (6,2 g) dans l'éther (100 ml), on ajoute 11 ml d'une solution d'éther chlorhydrique (2M dans l'éther) et agite 30 minutes. On concentre sous vide, reprend par de l'acétate d'éthyle (60 ml) et filtre le produit qui cristallise. De cette manière, on obtient 6,3 g du composé désiré.
Rendement : 95 % Fc : 115 °C
Spectre de R.M.N. ¹H (200 MHz, (DMSO), δ en ppm) ; 0,64 (t : 3H) ; 0,78 - 1,12 (m : 8H) ; 1,33 (qui : 2H) ; 1,66 (mt : 4H) ; 2,02 (mt : 2H) ; 2,20 (t : 2H) ; 2,74 (t : 2H) ; 2,85 - 3,15 (m : 6H) ; 6,50 (s : 1H) ; 6,92 (t : 1H) ; 7,20 (t : 1H) ; 7,30 - 7,57 (système AA'-BB' : 4H) ; 7,62 (d : 1H), 9,45 (d : 1H) ; 10,60 (se : 1H)

### Chlorhydrate hémihydrate de (2-butylindolizin-3-yl){4-[3-(dipropylamino)propyl]phényl}méthanone

A une solution de. (2-butylindolizin-3-yl){4-[3-(dipropylamino)propyl]phényl}méthanone (500 g) dans l'acétate d'éthyle (796 ml), on ajoute 11ml d'eau. Sur cette solution colorée refroidie à 10°C est ajoutée une solution d'éther chlorhydrique (2M dans l'éther). Le milieu est ensemencé puis maintenu trois heures en isotherme. Le produit obtenu est filtré, lavé avec du méthylisobutylcétone et séché
De cette manière, on obtient 478,5 g du composé désiré.
Rendement : 86,3 %
L'analyse RMN confirme la structure du composé.
DSC (« Differential Scanning Calorimetry »): Endotherme à 95,2°C correspondant à la perte d'eau, suivi d'un endotherme à 117,1°C correspondant à la fusion.

### Sulfate de (2-butylindolizin-3-yl){4-[3-(dipropylamino)propyl]phényl}méthanone

A une solution de (2-butylindolizin-3-yl){4-[3-(dipropylamino)propyl]phényl}méthanone (4,19 g) dans l'acétate d'éthyle (30 ml) puis chauffée à 70°C est ajoutée une solution d'acide sulfurique dans l'acétate d'éthyle. Le milieu est refroidi à 20°C puis après un isotherme à cette température, le composé obtenu est filtré, lavé et séché
De cette manière, on obtient 4,4 g du composé désiré.
Rendement : 85 %
L'analyse RMN confirme la structure du composé.
DSC: endotherme à 92°C correspondant à la fusion.

### Fumarate de (2-bulylindolizin-3-yl){4-[3-(dipropylamino)propyl]phényl}méthanone

A une solution de (2-butylindolizin-3-yl){4-[3-(dipropylamino)propyl]phényl}méthanone (9,5g) dans l'acétate d'éthyle (39 ml) puis chauffée à 50°C est ajoutée une solution d'acide fumarique dans l'acétate d'éthyle. Le milieu est refroidi à 30°C, ensemencé puis refroidi à 5°C. Après un isotherme à cette température le composé obtenu est filtré et séché. De cette manière, on obtient 9,2g du composé désiré.
Rendement : 75,8 %
L'analyse RMN confirme la structure du composé.
DSC: endotherme de fusion à 83,9°C

Suivant les procédés décrits ci-dessus dans les exemples 2 ou 3, mais en utilisant les produits de départ appropriés, on a préparé les composés ci après :
Chlorhydrate de (2-butylindolizin-3-yl){4-[3-(diéthylamino)propyl]phényl}méthanone
   Fc=145°C
   Spectre de R.M.N. ¹H (200 MHz, (DMSO), δ en ppm) ; 0,65 (t : 3H) ; 0,99 (sxt : 2H) ; 1,18 (t : 6H) ; 1,34 (qui : 2H) ;1,99 (mt : 2H) ; 2,20 (t : 2H) ; 2,75 (t : 2H) ; 2 ;93 - 3,22 (m : 6H) ; 6,50 (s : 1H) ; 6,92 (t : 1H) ; 7,20 (t : 1H); 7,32- 7,57 (système AA'-BB' : 4H) ; 7,62 (d : 1H) ; 9,45 (d : 1H) ; 10,10 (se: 1H)
Chlorhydrate de {4-[3-(diéthylamino)propyl]phényl}(2-propylindolizin-3-yl)méthanone
   Fc=152°C
   Spectre de R.M.N. ¹H (200 MHz, (DMSO), δen ppm) ; 0,60 (t : 3H) ; 1,19 - (t : 6H) ; 1,38 (sxt : 2H) ; 2,01 (mt : 2H) ; 2,17 (t : 2H) ; 2,75 (t : 2H) ; 2,89 - 3,21 (m : 6H) ; 6,51 (s : 1H) ; 6,92 (t : 1H) ;7,20 (t : 1H) ; 7,32 - 7,57 (système AA'-BB' : 4H) ; 7,62 (d : 1H) ; 9,45 (d : 1H)-10,52 (se : 1H)
Oxalate de (2-butylindolizin-3-yl){4-[4-(diéthylamino)butyl]phényl}méthanone
   Fc=102°C
   Spectre de R.M.N. ¹H (200 MHz, (DMSO), δ en ppm) ; 0,64 (t : 3H) ; 0,98 (sxt: 2H) ; 1,17 (t : 6H) ; 1,33 (qui : 2H) ; 1,65 (t : 4H) ; 2,20 (t : 2H) ; 2,70 (t : 2H) ; 2,93 - 3,21 (m : 6H) ; 6,50 (s : 1H) ; 6,92 (t : 1H) ; 7,20 (t : 1H) ; 7,29 - 7,56 (système AA'-BB' : 4H) ; 7,62 (d : 1H), 9,45 (d : 1H)
Oxalate de {4-[4-(diéthylamino)butyl]phényl}(2-éthylindolizin-3-yl)méthanone
   Fc=143°C
   Spectre de R.M.N. ¹H (200 MHz, (DMSO), δ en ppm) ; 0,98 (t : 3H) ; 1,15 (t: 6H) ; 1,64 mt : 4H) ; 2,20 (q: 2H) ; 2,72 (t : 2H) ; 2,89 - 3,19 (m : 6H) ; 6,53 (s : 1H) ; 6,92 (t : 1H) ; 7,20 (t : 1H) ; 7,29 -7,57 (système AA'-BB' : 4H) ; 7,63 (d : 1H); 9,45 (d :1H)
Chlorhydrate de {4-[4-(diéthylamino)butyl]phényl}(2-propylindolizin-3-yl)méthanone
   Fc=133°C
   Spectre de R.M.N. ¹H (200 MHz, (DMSO), δ en ppm) ; 0,60 (t : 3H) ; 1,20 (t : 6H) ; 1,37 (sxt : 2H) ;1,68 (mt : 4H) ; 2,17 (t : 2H) ; 2,71 (t : 2H) ; 2,90 - 3,18 (m : 6H) ; 6,50 (s : 1H) ; 6,93 (t: 1H) ;7,20 (t : 1H) ; 7,29 - 7,57 (système AA'-BB' : 4H) ; 7,62 (d : 1H) ; 9,46 (d : 1H) -10,37 (se 1H)
Chlorhydrate de {4-[3-(dipropylamino)propyl]phényl}(2-éthylindolizin-3-yl)méthanone
   Fc=152°C
   Spectre de R.M.N. ¹H (200 MHz, (DMSO), δ en ppm) ; 0,89 (t : 6H) ; 0,98 (t : 3H) ; 1,65 (mt : 4H) ; 2,03 (mt: 2H) ; 2,20 (q: 2H) ; 2,74 (t : 2H) ; 2,86 - 3,14 (m : 6H) ; 6,54 (s : 1H) ; 6,93 (t : 1H) ; 7,21 (t : 1H) ; 7,32 - 7,58 (système AA'-BB' : 4H) ; 7,63 (d : 1H) ; 9,47 (d : 1H) ; 10,46 (se : 1H)
Chlorhydrate de {4-[3-(dipropylamino)propyl]phényl}(2-propylindolizin-3-yl)méthanone
   Fc=122,6°C
   Spectre de R.M.N. ¹H (200 MHz, (DMSO), δ en ppm) ; 0,60 (t : 3H) ; 0,89 - (t : 6H) ; 1,3 (sx : 2H) ; 1,65 (m : 4H) ; 1,89 - 2,26 (m : 4H) ; 2,75 (t : 2H) ; 2,85 - 3,14 (m : 6H); 6,50 (s : 1H); 6,92 (t : 1H);7,20 (t : 1H); 7,32 - 7,57 (système AA-BB : 4H) ; 7,62 (d : 1H) ; 9,45 (d : 1H) -10,47 (singulet élargi : 1H)
Chlorhydrate de (2-butylindolizin-3-yl){4-[4-(dipropylamino)butyl]phényl}méthanone
   Fc=117°C
   Spectre de R.M.N. ¹H (250 MHz, (DMSO), δ en ppm) ; 0,64 (t : 3H) ; 0,79 - 1,08 (superposition de multiplets : 8H) ; 1,31 (qui : 2H) ; 1,48 - 1,89 (superposition de multiplets :8H) ; 2,18 (mt : 2H) ; 2,70 (mt : 2H) ; 2,78 - 3,17 (superposition de multiplets : 6H) ; 6,48 (s : 1H) ; 6,92 (t : 1H) ; 7,21 (t : 1H) ; 7,35 (d : 2H) ;7,48 (d : 2H) ; 7,60 (d : 1H) ; 9,45 (d : 1H) ; 10,56 (large signal : 1H)
Chlorhydrate de {4-[4-(dipropylamino)butyl]phényl}(2-éthylindolizin-3-yl)méthanone
   Fc=130°C
   Spectre de R.M.N. ¹H (200 MHz, (DMSO), δ en ppm) ; 0,89 (t : 6H) ; 0,98 (t : 3H) ; 1,67 (mt : 8H) ; 2,21 (q: 2H) ; 2,71 (t : 2H) ; 2,81 - 3,19 (m : 6H) ; 6,53 (s : 1H) ; 6,93 (t : 1H) ; 7,20 (t : 1H) ; 7,29 - 7,57 (système AA'-BB' : 4H) ; 7,63 (d : 1H) ; 9,45 (d : 1H) ; 10,32 (se : 1H)
Chlorhydrate de 4-[3-(dipropylamino)propyl]phényl}(2-isopropylindolizin-3-yl)méthanone
   Fc=154°C
   Spectre de R.M.N. ¹H (200 MHz, (DMSO-D₆), δ en ppm) ; 0,89 (t : 6H) ; 1,03 (d : 6H) ; 1,64 (mt : 4H) ; 2,03 (mt : 2H) ; 2,54 (mt : 1H) ; 2,75 (t : 2H) ; 2,84 - 3,17 (m : 6H) ; 6,58 (s : 1H) ; 6,89 (t : 1H) ; 7,17 (t : 1H) ; 7,39 (d : 2H) ; 7,55 (d : 2H) ; 7,62 (d : 1H); 9,32 (d : 1H) ; 10,54 (s : 1H)
Chlorhydrate de {4-[3-(dipropylamino)propyl]phényl}(2-méthylindolizin-3-yl)méthanone
   Fc=146°C
   Spectre de R.M.N. ¹H (200 MHz, (DMSO-D₆), δ en ppm) ; 0,87 (t : 6H) ; 1,62 (mt : 4H) ; 1,83 (s : 3H) ; 2,01 (mt : 2H) ; 2,17 (t : 2H) ; 2,84 - 3,12 (m : 6H) ; 6,46 (s : 1H) ; 6,94 (t :1H) ; 7,22 (t : 1H) ; 7,38 (d : 2H) ; 7,49 (d : 2H) ; 7,61 (d : 1H) ; 9,55 (d : 1H) ; 10,42 (s : 1H)
Oxalate de {4-[4-(dipropylamino)butyl]phényl}(2-isopropylindolizin-3-yl)méthanone
   Fc=64°C
   Spectre de R.M.N. ¹H (200 MHz, (DMSO-D₆), δ en ppm) ; 0,86 (t : 6H) ; 1,00 (d : 6H) ; 1,40 - 1,78 (m : 8H) ; 2,53 (mt : 1H) ; 2,69 (t : 2H) ; 2,81 - 3,15 (m : 6H) ; 6,58 (s : 1H) ; 6,87 (t : 1H); 7,16 (t : 1H) ; 7,34 (d : 2H) ; 7,54 (d : 2H) ; 7,60 (d : 1H) ; 9,30 (d : 1H)
Chlorhydrate de {4-[4-(dipropylamino)butyl]phényl}(2-methylindolizin-3-yl)méthanone
   Fc=141,1°C
   Spectre de R.M.N. ¹H (200 MHz, (DMSO-D₆), δ en ppm) ; 0,89 (t : 6H) ; 1,48 - 1,79 (m : 8H) ; 1,85 (s : 3H) ; 2,71 (t : 2H) ; 2,82 - 3,18 (m : 6H) ; 6,46 (s : 1H) ; 6,95 (t : 1H) ; 7,22 (t : 1H) ; 7,35 (d : 2H) ; 7,48 (d : 2H) ; 7,62 (d : 1H) ; 9,54 (d : 1H) ; 10,26 (s : 1H)
Chlorhydrate de (2-butylindolizin-3-yl){4-[3-(dibutylamino)propyl]phényl}méthanone
   Fc=83°C
   Spectre de R.M.N. ¹H (200 MHz, (DMSO), δ en ppm) ; 0,65 (t : 3H) ; 0,80 - 1,11 (m : 8H) ; 1,17 - 1,73 (m : 10H) ; 2,01 (mt :2H) ; 2,20 (t : 2H) ; 2,74 (t : 2H) ; 2,90 - 3,16 (m : 6H) ; 6,50 (s : 1H) ; 6,92 (t : 1H) ; 7,20 (t : 1H) ; 7,32 - 7,57 (système AA'BB' : 4H) ;7,62 (d : 1H) ; 9,45 (d : 1H) ; 10,25 (se : 1H)
Chlorhydrate de {4-[3-(dibutylamino)propyl]phényl}(2-éthylindolizin-3-yl)méthanone
   Fc=94,5°C
   Spectre de R.M.N. ¹H (200 MHz, (DMSO), δ en ppm) ; 0,90 (t : 6H) ; 0,99 (t : 3H) ; 1,30 (sxt: 4H) ; 1,61 (mt : 4H) ; 2,02 (mt : 2H) ; 2,21 (q : 2H) ; 2,75 (t : 2H) ; 2,87 - 3,18 (m : 6H) ; 6,55 (s : 1H) ; 6,94 (t : 1H) ; 7,22 (t : 1H) - 7,31 - 7,59 (système AA'-BB' : 4H) ; 7,64 (d : 1H) ; 9,45 (d : 1H) ; 10,48 (se : 1H)
Chlorhydrate de {4-[3-(dibutylamino)propyl]phényl}(2-propylindolizin-3-yl)méthanone
   Fc=120,6°C
   Spectre de R.M.N. ¹H (200 MHz, (DMSO), δ en ppm) ; 0,60 (t : 3H) ; 0,89 (t : 6H) ; 1,13-1,75 (m : 10H) ;1,87 - 1,25 (m : 4H) ; 2,74 (t : 2H) ; 2,88 - 3,15 (m : 6H) ; 6,50 (s : 1H) ; 6,93 (t : 1H) ; 7,20 (t: 1H) ;7,31 - 7,57 (système AA'-BB' : 4H) ; 7,62 (d : 1H) ; 9,45 (d : 1H) ; 10,44 (se : 1H)
Oxalate de (2-butylindolizin-3-yl){4-[4-(dibutylamino)butyl]phényl}méthanone
   Fc=101°C
   Spectre de R.M.N. ¹H (200 MHz, (DMSO), δ en ppm) ; 0,64 (t : 3H) ; 0,79 - 1,14 (superposition de multiplets : 8H) ; 1,14 - 1,45 (multiplets superposition de multiplets : 6H) ; 1,45 - 1,85 (multiplets superposition de multiplets : 8H) ; 2,20 (mt : 2H) ; 2,70 (mt : 2H) ; 2,87 - 3,20 (superposition de multiplets : 6H) ; 6,50 (s : 1H) ; 6,92 (t : 1H) ; 7,18 (t : 1H) ; 7,34 (d : 2H) ;7,49 (d : 2H) ; 7,61 (d : 1H) ; 9,44 (d : 1H)
Oxalate de {4-[4-(dibutylamino)butyl]phényl}(2-éthylindolizin-3-yl)méthanone
   Fc=96-101 °C
   Spectre de R.M.N. ¹H (200 MHz, (DMSO), δ en ppm) ; 0,89 (t : 6H) ; 0,98 (t : 3H) ; 1,30 (sxt : 4H) ; 1,43 -1,79 (m : 8H) ; 2,21 (q : 2H) ; 2,72 (t : 2H) ; 2,82 - 3,14 (m : 6H) ; 6,53 (s : 1H) ; 6,92 (t : 1H) ; 7,20 (t : 1H) ; 7,29 - 7,57 (système AA'-BB' : 4H) ; 7,63 (d : 1H) ; 9,45 (d : 1H)
Oxalate de {4-[4-(dibutylamino)butyl]phényl}(2-propylindolizin-3-yl)méthanone
   Fc=154,6°C
   Spectre de R.M.N. ¹H (200 MHz, (DMSO), δ en ppm) ; 0,60 (t : 3H) ; 0,90 (t : 6H) ; 1,12 - 1,82 (m : 14H) ;2,17 (t : 2H) ; 2,71 (t : 2H) 2,84 - 3,18 (m : 6H) ; 6,50 (s : 1H) ; 6,93 (t : 1H) ; 7,20 (t : 1H) ; 7,29 - 7,57 (système AA'-BB' : 4H) ; 7,62 (d : 1H) - 9,45 (d : 1H)
Chlorhydrate de (2-butylindolizin-3-yl)(4-{3-[éthyl(propyl)amino]propyl}phényl)méthanone
   Fc=120°C
   Spectre de R.M.N. ¹H (200 MHz, (DMSO), δ en ppm) ; 0,65 (t : 3H) ; 0,81 - 1,10 (m : 5H) ; 1,11 - 1,45 (m : 5H) ; 1,65 (mt: 2H) ; 2,01 (mt: 2H) ; 2,20 (t : 2H) ; 2,74 (t : 2H) ; 2,86 - 3,21 (m : 6H) ; 6,50 (s : 1H) ; 6,92 (t : 1H) ; 7,20 (t : 1H) ; 7,31 - 7,57 (système AA'-BB' :4H) ; 7,62 (d : 1H), 9,45 (d : 1H) ; 10,53 (se : 1H)
Oxalate de (2-éthylindolizin-3-yl)(4-{3-[éthyl(propyl)amino]propyl}phényl)méthanone
   Fc=160°C
   Spectre de R.M.N. ¹H (200 MHz, (DMSO), δ en ppm) ; 0,89 (t : 3H) ; 0,98 (t : 3H) ; 1,15 (t : 3H) ; 1,56 (mt: 2H) ; 1,97 (mt: 2H) ; 2,20 (q : 2H) ; 2,73 (t : 2H) ;2,86 - 3,19 (m : 6H) ; 6,54 (s : 1H) ; 6,93 (t : 4H) ; 7,21 (t : 1H) ; 7,30 - 7,58 (système AA'-BB' : 4H) ; 7,63 (d : 1H) 9,47 (d : 1H) ; 5,40 - 7,31 (se: 2H)
Chlorhydrate de (4-{3-[éthyl(propyl)amino]propyl}phényl)(2-isopropylindolizin-3-yl)méthanone
   Fc=130°C
   Spectre de R.M.N. ¹H (200 MHz, (DMSO-D₆), δ en ppm) ; 0,87 (t : 3H) ; 1,00 (d : 6H) ; 1,16 (t : 3H) ; 1,62 (mt : 2H) ; 2,00 (mt : 2H) ; 2,53 (mt : 1H) ; 2,73 (t : 2H) ; 2,84 - 3,17 (m : 6H) ; 6,57 (s : 1H) ; 6,88 (t : 1H); 7,16 (t : 1H) ; 7,38 (d : 2H) ; 7,55 (d : 2H) ; 7,61 (d : 1H) ;9,32 (d : 1H); 10,48 (s : 1H)
Chlorhydrate de (4-{3-[éthyl(propyl)amino]propyl}phényl)(2-méthylindolizin-3-yl)méthanone
   Fc=147,9°C
   Spectre de R.M.N.¹H (200 MHz, (DMSO), δ en ppm) ; 0,89 (t : 3H) ; 0,98 (t : 3H) ; 1,15 (t : 3H) ; 1,56 (m : 2H) ; 1,97 (m : 2H) ; 2,20 (q : 2H) ; 2,73 (t : 2H) ;2,86 - 3,19 (m : 6H) ; 6,54 (s : 1H) ; 6,93 (t : 4H) ; 7,21 (t : 1H) ; 7,30 - 7,58 (système AA'-BB' : 4H) ; 7,63 (d : 1H) 9,47 (d : 1H) ; 5,40 - 7,31 (signal élargi : 2H)
Oxalate de (4-{4-[éthyl(propyl)amino]butyl}phényl)(2-isopropylindolizin-3-yl)méthanone
   Fc=136°C
   Spectre de R.M.N. ¹H (200 MHz, (DMSO-D₆), δ en ppm) ; 0,89 (t : 3H) ; 1,03 (d : 6H) ; 1,17 (t : 3H) ; 1,47 - 1,80 (m ; 6H) ; 2,55 (mt : 1H) ; 2,72 (t : 2H) ; 2,84 - 3,19 (m : 6H) ; 6,58 (s : 1H) ; 6,88 (t: 1H); 7,17 (t: 1H); 7,35 (d : 2H) ; 7,54 (d : 2H) ; 7,62 (d : 1H) ; 9,31 (d : 1H) ; 5,50 - 8,00 (se : 2H)
Chlorhydrate de (4-{4-[éthyl(propyl)amino]butyl}phényl)(2-méthylindolizin-3-yl)méthanone
   Fc=141,1°C
   Spectre de R.M.N.¹H (200 MHz, (DMSO-D₆), δ en ppm) ; 0,89 (t : 3H) ; 1,20 (t : 3H) ; 1,43 - 1,79 (m : 6H) ; 1,86 (s : 3H) ; 2,71 (t : 2H) ; 2,82 - 3,22 (m : 6H) ; 6,46 (s : 1H) ; 6,95 (t : 1H) ; 7,22 (t : 1H) ; 7,35 (d : 2H) ; 7,48 (d : 2H) ; 7,62 (d : 1H) ; 9,55 (d : 1H) ; 10,30 (s : 1H)
Chlorhydrate de (4-{3-[éthyl(propyl)amino]propyl}phényl)(2-propylindolizin-3-yl)méthanone
   Fc=108,7°C
   Spectre de R.M.N. ¹H (200 MHz, (DMSO), δ en ppm) ; 0,60 (t : 3H) ; 0,89 - (t : 3H) ; 1,19 (t : 3H) ;1,38 (sxt : 2H) ; 1,64 (mt : 2H) ; 1,89 -2,26 (m : 4H) ; 2,75 (t : 2H) ; 2,85 - 3,21 (m : 6H) ; 6,51 (s : 1H) ;6,93 (t : 1H) ; 7,20 (t : 4H) ; 7,31 - 7,58 (système AA'-BB' : 4H) ; 7,62 (d : 1H) -9,45 (d : 1H) -10,38 (se : 1H))
Chlorhydrate de (2-butylindolizin-3-yl)(4-{4-[éthyl(propyl)amino]butyl}phényl)méthanone
   Fc=125°C
   Spectre de R.M.N. ¹H (200 MHz, (DMSO), δ en ppm) ; 0,63 (t : 3H) ; 0,77 - 1,10 - (m : 5H) ; 1,11 - 1,45 (m : 5H) ; 1,67 (mt : 6H) ; 2,19 (t : 2H) ; 2,70 (t : 2H) ; 2,82 - 3,21 (m : 6H) ; 6,50 (s : 1H) ; 6,91 (t : 1H) ;7,19 (t : 1H) ; 7,29 - 7,55 (système AA'-BB' : 4H) ; 7,61 (d : 1H) ; 9,45 (d : 1H) -10,55 (se : 1H)
Oxalate de (2-éthylindolizin-3-yl)(4-{4-[éthyl(propyl)amino]butyl}phényl)méthanone
   Fc=132°C
   Spectre de R.M.N. ¹H (200 MHz, (DMSO), δ en ppm) ; 0,72 - 1,35 (m : 9H) ; 1,65 - (se : 6H) ; 2,20 (q : 2H) ; 2,70 (se : 2H) ; 2,82 - 3,21 (m : 6H) ; 6,54 (s : 1H) ; 6,93 (t : 1H) ; 7,20 (t : 1H) ; 7,27 - 7,57 (système AA'-BB' : 4H) ;7,63 (d : 1H) ; 7,63 (d : 1H) ; 9,45 (d : 1H) ; 8,20 - 10 (se : 1H)
Chlorhydrate de (4-{4-[éthyl(propyl)amino]butyl}phényl)(2-propylindolizin-3-yl)méthanone
   Fc=133°C
   Spectre de R.M.N. ¹H (200 MHz, (DMSO), δ en ppm) ; 0,57 (t : 3H) ; 0,87 (t : 3H) ; 1,18 (t : 3H) ; 1,35 (sxt : 2H) ; 1,65 (m : 6H) ; 2,14 (t : 2H) ; 2,69 (t : 2H) ; 2,80 - 3,18 (m : 6H) ; 6,49 (s : 1H) ; 6,91 (t : 1H); 7,19 (t : 1H) ; 7,27- 7,56 (système AA'-BB' : 4H) ; 7,61 (d : 1H) ; 9,45 (d : 1H) ; 10,28 (se : 1H)
Oxalate de (2-butylindolizin-3-yl)(4-{3-[butyl(propyl)amino]propyl}phényl)méthanone
   Fc=142°C
   Spectre de R.M.N. ¹H (200 MHz, (DMSO), δ en ppm) ; 0,64 (t : 3H) ; 0,79 - 1,13 (superposition de multiplets : 8H) ; 1,14 - 1,45 (superposition de multiplets : 4H) ; 1,45 -1,75 (superposition de multiplets : 4H) ; 1,96 (mt : 2H) ; 2,20 (mt : 2H) ; 2,73 (mt : 2H) ; 2,86 - 3,17 (superposition de multiplets : 6H) ; 6,51 (s : 1H) ; 6,92 (t : 1H) ; 7,20 (t : 1H) ;7,38 (d : 2H) ; 7,52 (d : 2H) ; 7,62 (d : 1H); 9,46 (d : 1H)
Oxalate de (4-{3-[butyl(éthyl)amino]propyl}phényl)(2-butylindolizin-3-yl)méthanone
   Fc=112°C
   Spectre de R.M.N. ¹H (200 MHz, (DMSO), δ en ppm) ; 0,64 (t : 3H) ; 0,79 - 1,08 (superposition de multiplets : 5H) ; 1,15 (t 3H) ; 1,23-1,44 (superposition de multiplets : 4H) ; 1,55 (mt : 2H) ; 1,97 (mt : 2H) ; 2,20 (mt : 2H) ; 2,74 (t : 2H) ; 2,90-3,20 (superposition de multiplets : 6H) ; 6,51 (s : 1H) ; 6,92 (t : 1H) ;7,21 (t : 1H) ; 7,39 (d : 2H) ; 7,52 (d : 2H) ; 7,62 (d : 1H) ; 9,46 (d : 1H)
Chlorhydrate de (2-butylindolizin-3-yl)(4-{3-[(cyclopropylméthyl)(propyl)amino]propyl}phényl)méthanone
   Fc=101°C
   Spectre de R.M.N. ¹H (200 MHz, (DMSO), δ en ppm) ; 0,34 (mt : 2H) ; 0,50 - 0,80 (superposition de multiplets : 5H) ; 0,80 - 1,17 (superposition de multiplets : 6H) ; 1,33 (qui : 2H) ; 1,63 (mt : 2H) ; 2,00 (mt : 2H) ; 2,20 (mt : 2H) ; 2,73 (t : 2H) ; 2,86 - 3,40 (superposition de multiplets : 6H) ; 6,51 (s : 1H) ; 6,95 (t : 1H) ; 7,19 (t : 1H) ; 7,38 (d : 2H) ; 7,52 (d : 2H) ; 7,60 (d : 1H) ; 9,45 (d : 1H)
Chlorhydrate de {4-[4-(propylamino)butyl]phényl}(2-propylindolizin-3-yl)méthanone
   Fc=135,3°C
   Spectre de R.M.N. ¹H (200 MHz, (DMSO-D₆), δ en ppm) ; 0,59 (t : 3H) ; 0,90 (t : 3H) ; 1,37 (sxt : 2H) ; 1,50 - 1,80 (m : 6H) ; 2,16 (t : 2H) ; 2,57 - 3,04 (m : 6H) ; 6,50 (s : 1H) ; 6,92 (t : 1H); 7,20 (t : 1H) ; 7,35 (d : 2H) ; 7,50 (d : 2H) ; 7,62 (d : 1H) ; 8,92 (s : 2H) ; 9,45 (d : 1H)
Chlorhydrate de (2-butylindolizin-3-yl){4-[3-(propylamino)propyl]phényl}méthanone
   Fc=178,2°C
   Spectre de R.M.N. ¹H (200 MHz, (DMSO-D₆), δ en ppm) ; 0,62 (t : 3H) ; 0,78 - 1,07 (m : 5H) ; 1,31 (qui : 2H) ; 1,62 (sxt : 2H) ; 1,96 (qui : 2H) ; 2,17 (t : 2H) ; 2,61 - 2,99 (m : 6H) ; 6,49 (s : 1H) ; 6,91 (t : 1H) ; 7,20 (t : 1H) ; 7,36 (d : 2H) ; 7,51 (d : 2H) ; 7,61 (d : 1H) ; 8,99 (s : 2H) ; 9,46 (d : 1H)
Chlorhydrate de (2-butylindolizin-3-yl){4-[3-(diméthylamino)propyf]phényl}méthanone
   Fc=133,6°C
   Spectre de R.M.N. ¹H (200 MHz, (DMSO-D₆), δ en ppm) ; 0,64 (t : 3H) ; 0,99 (sxt : 2H) ; 1,34 (qui : 2H) ; 2,02 (m : 2H) ; 2,20 (t : 2H) ; 2,59 - 2,87 (m : 8H) ; 3,04 (m : 2H) ; 6,50 (s : 1H) ; 6,92 (t : 1H) ; 7,20 (t : 1H) ;7,37 (d : 2H) ; 7,51 (d : 2H) ; 7,62 (d : 1H) ; 9,46 (d : 1H) ; 10,78 (s : 1H)
Chlorhydrate de (2-butylindolizin-3-yl)(4-{3-[(3R,5S)-3,5-dimethylpipérazin-1-yl]propyl}phényl)méthanone
   MH+=432,3
   Spectre de R.M.N. ¹H (200 MHz, (DMSO), δ en ppm) ; 0,65 (t : 3H) ; 0,98 (mt : 2H) ; 1,17 - 1,48 (superposition multiplets : 8H) ; 1,93 - 2,32 (superposition multiplets :4H) ; 2,75 (t : 2H) ; 3,13 (mt : 4H) ; 3,73 (mt : 4H) ; 6,51 (s : 1H) ; 6,92 (t : 1H) ; 7,21 (t : 1H) ; 7,39 (d : 2H) ;7,53 (d : 2H) ; 7,63 (d : 1H) ; 9,45 (d : 1H) ; 9,96 (signal large : 2H) ; 12,06 (signal large : 1H)
Chlorhydrate de (2-butylindolizin-3-yl)(4-{3-[(3*R*,5*S*)-3,4,5-triméthylpipérazin-1-yl]propyl}phényl)méthanone
   Fc=191-201°C
   Spectre de R.M.N. ¹H (200 MHz, (DMSO), δ en ppm) ; 0,65 (t : 3H) ; 0,98 (mt: 2H) ; 1,15 - 1,58 (superposition multiplets : 8H) ; 1,93 - 2,38 (superposition multiplets : 4H) ; 2,50 - 2,90 (superposition multiplets : 5H) ; 2,95 - 4,50 (superposition multiplets : 8H) ; 6,55 (s : 1H) ; 6,92 (t : 1H) ; 7,21 (t : 1H) ; 7,39 (d : 2H) ; 7,53 (d : 2H) ;7,63 (d : 1H); 9,45 (d : 1H); 11,95 (signal large : 2H)
Oxalate de (4-{3-[bis(2-méthoxyéthyl)amino]propyl}phényl)(2-butylindolizin-3-yl)méthanone
   Fc=137-139°C
   Spectre de R.M.N. ¹H (200 MHz, (DMSO), δ en ppm) ; 0,65 (t : 3H) ; 0,98 (mt : 2H) ; 1,33 (mt : 2H) ; 1,90 (mt : 2H) ; 2,19 (mt : 2H) ; 2,69 (mt : 2H) ; 2,95 (mt : 2H) ; 3,12 (mt : 4H) ; 3,26 (s : 6H) ; 3,55 (mt : 4H) ; 6,51 (s : 1H) ;6,92 (t : 1H) ; 7,20 (t : 1H) ; 7,34 (d : 2H); 7,51 (d : 2H) ; 7,63 (d : 1H) ; 9,46 (d : 1H)
Oxalate de (2-butylindolizin-3-yl)[4-(3-pipéridin-1-ylpropyl)phényl]méthanone
   Fc=191-192°C
   Spectre de R.M.N. ¹H (200 MHz, (DMSO), δ en ppm) ; 0,65 (t : 3H) ; 0,98 (mt : 2H) ; 1,33 (mt : 2H) ; 1,53 (mt :2H) ; 1,71 (superposition de multiplets : 4H) ; 2,00 (mt : 2H) ; 2,20 (mt : 2H) ; 2,70 (t : 2H) ; 2,88 - 3,30 (superposition de multiplets : 6H) ; 6,52 (s : 1H) ; 6,92 (t : 1H) ;7,28 (t : 1H) ; 7,37 (d : 2H) ; 7,52 (d : 2H); 7,63 (d : 1H) ; 9,45 (d : 1H)

### Exemple 4 Chlorhydrate de {4-[4-(dipropylamino)butyl]phényl}(2-propylindolizin-3-yl)méthanone (composé 16)

### a) (2-propylindolizin-3-yl-(4-chlorobutyl)phényl]méthanone

On chauffe à 50°C pendant 18 heures un mélange de 11 g (69 mmoles) de 2-propyl indolizine et de 15,9 g (69 mmoles) de chlorure de 4-(4-chlorobutyl) benzoyle. On reprend par de l'eau, extrait au dichlorométhane, lave avec une solution de carbonate de sodium, sèche la phase organique sur sulfate de sodium et concentre à sec. On purifie alors par flash chromatographie sur alumine (éluant : heptane/ Chlorure de méthylène 50/50).
De cette manière on obtient 23 g de composé désiré sous la forme d'une huile.
Rendement: 94,2 %

### b) (2-propylindolizin-3-yl){4-[4-(dipropylamino)butyl]phényl}méthanone

Dans un réacteur, on dissout 4 g (11,3 mmoles) du composé obtenu à l'étape précédente dans 50 ml de méthyl isobutylcétone et on ajoute à ce milieu réactionnel 2,9 g (29,3 mmoles) de dipropylamine, 0,33 g (2,26 mmoles) d'iodure de sodium et chauffe à 110°C pendant 72 heures.
On verse dans l'eau, extrait au dichlorométhane, lave avec une solution d'hydrogénocarbonate de sodium et concentre à sec. On purifie alors par flash chromotographie sur silice (éluant : Chlorure de méthylène /méthanol 95/5)
De cette manière on obtient 3,9g du composé désiré.
Rendement : 82,9%

### c) Chlorhydrate de (2-propylindolizin-3-yl){4-[4-(dipropylamino)butyl]phényl}méthanone

A une solution du produit décrit précédemment 3,9 g dans l'éther (50 ml), on ajoute 7 ml d'une solution d'éther chlorhydrique (2 M dans l'éther), agite 30 minutes et filtre le sel qui cristallise.
De cette manière on obtient 3,1g du composé désiré.
Rendement : 73%
Fc = 93°c
Spectre de R.M.N. ¹H (200 MHz, (DMSO), δ en ppm) ; 0,60 (t : 3H) ; 0,90 (t : 6H) ; 1,38 (sxt : 2H) ;1,52 - 1,85 (m : 8H) ; 2,17 (t : 2H) 2,71 (t : 2H) ; 2,84 - 3,18 (m : 6H) ; 6,50 (s : 1H) ; 6,93 (t: 1H) ;7,20 (t : 1H) ; 7,29 - 7,57 (système AA'-BB' : 4H) ; 7,62 (d : 1H) ; 9,45 (d : 1H) -10,23 (se : 1H)

Les tests de solubilités ont été effectués à pH ≥4 de la façon suivante.
On prépare un tampon phosphate pH 6 0,1M, par addition de 6,15 ml Na₂HPO₄ 0,1M avec 43,85 ml NaH₂PO₄ 0,1M. La solution est complétée à 100 ml avec de l'eau filtrée au moyen d'un système Millipore (marque déposée).

### Référence (produit à tester à une concentration où ce dernier est soluble et qui sert de référence interne)

On pèse exactement environ 0,2 mg (dans une nacelle) et on le dilue dans 1 ml H₂O/CH₃CN: (50:50) soit 0,2 mg/ml puis on passe l'échantillon 5 minutes aux ultrasons.

### Echantillon du produit à tester:

On pèse exactement environ 4 mg (dans une nacelle) et on le dilue dans 400 µl (soit 10 mg/ml) de tampon phosphate pH 6 (0,1 M) puis on passe l'échantillon 5 minutes aux ultrasons.
- on note le pH et si le pH est inférieur à 4, on ajoute 1 µl de NaOH 1 N (de 1 µl en 1 µl) jusqu'à pH supérieur à 4 puis on note à nouveau le pH. On filtre la solution (0,45 µm) puis on dilue si nécessaire avec le tampon phosphate pH6. (Si le produit reprécipite après dilution, on ne tient pas compte de l'échantillon dilué, on reprend la solution mère et on détermine la solubilité à une longueur d'onde différente du λ max. pour éviter la saturation du signal.
On détermine ensuite la solubilité du composé par HPLC dans les conditions suivantes :

### Conditions expérimentales

Instrument : Chromatographe Agilent 1100
Logiciel : Chemstation
Colonne : XTERRA C18 (150 x 4,6 mm ; 3,5 µm)
Eluant A : H₂O / CH₃CN / CH₃SO₃H : 1000 / 25 / 1
Eluant B : H₂O / CH₃CN / CH₃SO₃ 25 / 1000 / 1
Gradient :

| Temps (min) | A | B |
|---|---|---|
| 0 | 90 | 10 |
| 8 | 0 | 100 |
| 16 | 0 | 100 |
| 17-20 | 90 | 10 |

Température colonne : 30°C
Débit : 0,8 ml/min
Détection : λ= 230 nm
Volume d'injection : 5 µL

## Revendications

1. Dérivés d'indolizine de formule générale (I) 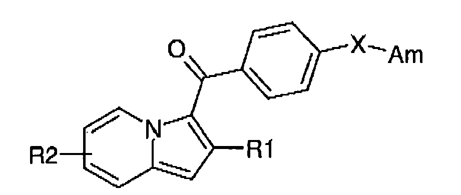 dans laquelle,
X représente un radical -(CH₂)n- avec n étant un nombre entier allant de 1 à 6,
R₁ représente un radical alkyle en C₁-C₈ linéaire, ramifié ou cyclique ;
Am représente un groupe NR₃R₄ dans lequel R₃ et R₄ sont identiques ou différents et représentent indépendamment l'un de l'autre
- un atome d'hydrogène,
- un radical alkyle en C₁-C₆ linéaire, ramifié ou cyclique, ou en C₁-C₃ substitué par un radical cycloalkyle en C₃-C₆,
- ou un radical (CH₂)I-O-B, dans lequel B représente un atome d'hydrogène ou un radical alkyle (CH₂)k, avec I est k étant des nombres entiers I ≥ 2 et I+k ≤ 6
ou R₃ et R₄ forment ensemble avec l'atome d'azote auquel ils sont attachés un hétérocycle de 4 à 6 chaînons pouvant éventuellement contenir un ou plusieurs hétéroatomes choisis parmi N, O et portant éventuellement un ou plusieurs substituants choisis parmi un radical alkyle en C₁-C₆ linéaire, ramifié ou cyclique,
R₂ représente un atome d'hydrogène, un radical alkyle en C₁-C₆ linéaire, ramifié ou cyclique,
et leurs sels pharmaceutiquement acceptables.

2. Dérivés d'indolizine selon la revendication 1 **caractérisé en ce que** X représente un radical (CH₂)n- avec n étant un nombre entier allant de 3 à 4.

3. Dérivés d'indolizine selon la revendication 1, 2 ou 3 **caractérisé en ce que** R₁ représente un radical alkyle en C₁-C₄.

4. Dérivés d'indolizine selon l'une quelconque des revendications 1 à 4 **caractérisé en ce que** R₃ et R₄ représentent indépendamment l'un de l'autre un radical alkyle en C₁-C₄ linéaire,

5. Dérivés d'indolizine selon l'une quelconque des revendications 1 à 5 **caractérisé en ce que** R₃ et R₄ forment ensemble un groupement pipéridinyle ou pipérazinyle portant éventuellement un ou plusieurs radical méthyl.

6. Dérivés d'indolizine selon l'une quelconque des revendications 1 à 6 **caractérisé en ce que** R₂ représente un atome d'hydrogène.

7. Dérivés d'indolizine selon l'une quelconque des revendications 1 à 7 **caractérisé en ce que** le sel pharmaceutiquement acceptable est choisi parmi l'oxalate et le chlorhydrate.

8. Dérivés d'indolizine de formule (I) selon la revendication 1 dont les noms suivent :
Chlorhydrate de (2-butylindolizin-3-yl){4-[3-(diéthylamino)propyl]phényl}méthanone
Oxalate de (2-éthylindolizin-3-yl){4-[3-(diéthylamino)propyl]phényl}méthanone Chlorhydrate de {4-[3-(diéthylamino)propyl]phényl}(2-propylindolizin-3-yl)méthanone
Oxalate de (2-butylindolizin-3-yl){4-[4-(diéthylamino)butyl]phényl}méthanone
Oxalate de {4-[4-(diéthylamino)butyl]phényl}(2-éthylindolizin-3-yl)méthanone
Chlorhydrate de {4-[4-(diéthylamino)butyl]phényl}(2-propylindolizin-3-yl)méthanone
Chlorhydrate de (2-butylindolizin-3-yl){4-[3-(dipropylamino)propyl]phényl}méthanone
Chlorhydrate hémihydrate de (2-butylindolizin-3-yl){4-[3-(dipropylamino)propyl]phényl}méthanone
Sulfate de (2-butylindolizin-3-yl){4-[3-(dipropylamino)propyl]phényl}méthanone
Fumarate de (2-butylindolizin-3-yl){4-[3-(dipropylamino)propyl]phényl}méthanone
Chlorhydrate de {4-[3-(dipropylamino)propyl]phényl}(2-éthylindolizin-3-yl)méthanone
Chlorhydrate de {4-[3-(dipropylamino)propyl]phényl}(2-propylindolizin-3-yl)méthanone
Chlorhydrate de (2-butylindolizin-3-yl){4-[4-(dipropylamino)butyl]phényl}méthanone
Chlorhydrate de {4-[4-(dipropylamino)butyl]phényl}(2-éthylindolizin-3-yl)méthanone
Chlorhydrate de 4-[3-(dipropylamino)propyl]phényl}(2-isopropylindolizin-3-yl)méthanone
Chlorhydrate de {4-[3-(dipropylamino)propyl]phényl}(2-méthylindolizin-3-yl)méthanone
Oxalate de {4-[4-(dipropylamino)butyl]phényl}(2-isopropylindolizin-3-yl)méthanone
Chlorhydrate de {4-[4-(dipropylamino)butyl]phényl}(2-méthylindolizin-3-yl)méthanone
Chlorhydrate de {4-[4-(dipropylamino)butyl]phényl}(2-propylindolizin-3-yl)méthanone
Chlorhydrate de (2-butylindolizin-3-yl){4-[3-(dibutylamino)propyl]phényl}méthanone
Chlorhydrate de {4-[3-(dibutylamino)propyl]phényl}(2-éthylindolizin-3-yl)méthanone
Chlorhydrate de {4-[3-(dibutylamino)propyl]phényl}(2-propylindolizin-3-yl)méthanone
Oxalate de (2-butylindolizin-3-yl){4-[4-(dibutylamino)butyl]phényl}méthanone
Oxalate de {4-[4-(dibutylamino)butyl]phényl}(2-éthylindolizin-3-yl)méthanone
Oxalate de {4-[4-(dibutylamino)butyl]phényl}(2-propylindolizin-3-yl)méthanone
Chlorhydrate de (2-butylindolizin-3-yl)(4-{3-[éthyl(propyl)amino]propyl}phényl)méthanone
Oxalate de (2-éthylindolizin-3-yl)(4-{3-[éthyl(propyl)amino]propyl}phényl)méthanone
Chlorhydrate de (4-{3-[éthyl(propyl)amino]propyl}phényl)(2-isopropylindolizin-3-yl)méthanone
Chlorhydrate de (4-{3-[éthyl(propyl)amino]propyl}phényl)(2-méthylindolizin-3-yl)méthanone
Oxalate de (4-{4-[éthyl(propyl)amino]butyl}phényl)(2-isopropylindolizin-3-yl)méthanone
Chlorhydrate de (4-{4-[éthyl(propyl)amino]butyl}phényl)(2-méthylindolizin-3-yl)méthanone
Chlorhydrate de (4-{3-[éthyl(propyl)amino]propyl}phényl)(2-propylindolizin-3-yl)méthanone
Chlorhydrate de (2-butylindolizin-3-yl)(4-{4-[éthyl(propyl)amino]butyl}phényl)méthanone
Oxalate de (2-éthylindolizin-3-yl)(4-{4-[éthyl(propyl)amino]butyl}phényl)méthanone
Chlorhydrate de (4-{4-[éthyl(propyl)amino]butyl}phényl)(2-propylindolizin-3-yl)méthanone
Oxalate de (2-butylindolizin-3-yl)(4-{3-[butyl(propyl)amino]propyl}phényl)méthanone
Oxalate de (4-{3-[butyl(éthyl)amino]propyl}phényl)(2-butylindolizin-3-yl)méthanone
Chlorhydrate de (2-butylindolizin-3-yl)(4-{3-[(cyclopropylméthyl)(propyl)amino]propyl}phényl)méthanone
Chlorhydrate de {4-[4-(propylamino)butyl]phényl}(2-propylindolizin-3-yl)méthanone
Chlorhydrate de (2-butylindolizin-3-yl){4-[3-(propylamino)propyl]phényl}méthanone
Chlorhydrate de (2-butylindolizin-3-yl){4-[3-(diméthylamino)propyl]phényl}méthanone
Chlorhydrate de (2-butylindolizin-3-yl)(4-{3-[(3*R*,5*S*)-3,5-diméthylpipérazin-1-yl]propyl}phényl)méthanone
Chlorhydrate de (2-butylindolizin-3-yl)(4-{3-[(3*R*,5*S*)-3,4,5-triméthylpipérazin-1-yl]propyl}phényl)méthanone
Oxalate de (4-{3-[bis(2-méthoxyéthyl)amino]propyl}phényl)(2-butylindolizin-3-yl)méthanone
Oxalate de (2-butylindolizin-3-yl)[4-(3-pipéridin-1-ylpropyl)phényl]méthanone
Chlorhydrate de (2-butyl-6-méthylindolizin-3-yl){4-[3-(dipropylamino)propyl]phényl}méthanone
Chlorhydrate de (2-butyl-6-éthylindolizin-3-yl){4-[3-(dipropylamino)propyl]phényl}méthanone
Chlorhydrate de (2-butyl-6-méthylindolizin-3-yl)(4-{3-[éthyl(propyl)amino]propyl}phényl)méthanone
Chlorhydrate de (2-butyl-6-éthylindolizin-3-yl)(4-{3-[éthyl(propyl)amino]propyl}phényl)méthanone

9. Procédé de préparation des dérivés de formule (I) telle que définie dans l'une quelconque des revendications 1 à 4 comprenant la réaction d'une amine H-Am sur un composé représenté par la formule (II) : 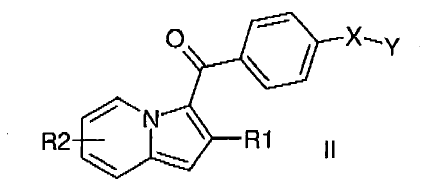 dans laquelle Y représente un atome d'halogène et les autres substituants sont tels que définis dans les revendications précédentes.

10. Procédé comprenant un procédé de préparation du composé de formule (II) selon la revendication 9 et comprenant la réaction d'un composé représenté par la formule (III) : 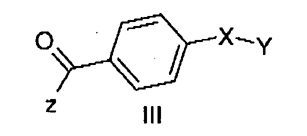 sur un composé représenté par la formule (IV):

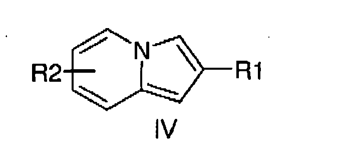

11. Dérivés d'indolizine de formule générale (I) tels que définis à l'une quelconque des revendications 1 à 9 pour leur utilisation en tant que médicament.

12. Médicaments comprenant les dérivés d'indolizine de formule générale (I) tels que définis à l'une quelconque des revendications 1 à 8.

13. Composition pharmaceutique ou vétérinaire, **caractérisée en ce qu'**elle contient comme principe actif au moins un dérivé d'indolizine de formule (I) selon l'une quelconque des revendications 1 à 8, ou un sel pharmaceutiquement acceptable de ce dernier, en association avec un véhicule pharmaceutique ou un excipient approprié.

14. Utilisation d'un dérivé d'indolizine de formule générale (I) tels que définis à l'une quelconque des revendications 1 à 8 pour la préparation d'un médicament destiné au traitement des syndromes pathologiques du système cardio-vasculaire en particulier dans le traitement de l'angine de poitrine, de l'hypertension, de l'arythmie ventriculaire ou supraventriculaire, le traitement de l'insuffisance cardiaque, de l'infarctus du myocarde compliqué ou non d'insuffisance cardiaque ou pour la prévention de la mortalité post-infarctus.

15. Utilisation d'un dérivé d'indolizine selon la revendication 14 pour la préparation d'un médicament destiné au traitement de l'angine de poitrine, de l'hypertension, de l'arythmie ventriculaire ou supraventriculaire, le traitement de l'insuffisance cardiaque, de l'infarctus du myocarde compliqué ou non d'insuffisance cardiaque ou pour la prévention de la mortalité post-infarctus.

## Claims

1. Indolizine derivatives with general formula (I): 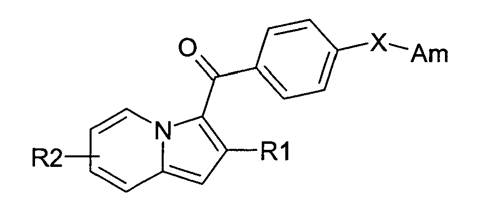 in which:
X represents a - (CH₂)ₙ- radical where n is a whole number from 1 to 6,
R₁ represents a linear, branched or cyclic C₁-C₈ alkyl radical;
Am represents an NR₃R₄ group in which R₃ and R₄ are identical or different and independently of each other represent:
- a hydrogen atom;
- a linear, branched or cyclic C₁-C₆ alkyl radical or a C₁-C₃ radical substituted with a C₃-C₆ cycloalkyl radical;
- or a (CH₂)₁-O-B radical in which B represents a hydrogen atom or a (CH₂)ₖ alkyl radical, where 1 and k are whole numbers, 1 ≥ 2 and 1+k = 6;
or R₃ and R₄ together with the nitrogen atom to which they are attached form a heterocycle containing 4 to 6 links which may optionally contain one or more heteroatoms selected from N and O and optionally carrying one or more substituents selected from a linear, branched or cyclic C₁-C₆ alkyl radical;
R₂ represents a hydrogen atom or a linear, branched or cyclic C₁-C₆ alkyl radical;
and their pharmaceutically acceptable salts.

2. Indolizine derivatives according to Claim 1, **characterized in that** X represents a (CH₂)ₙ-radical where n is a whole number from 3 to 4.

3. Indolizine derivatives according to Claim 1 or 2, **characterized in that** R₁ represents a C₁-C₄ alkyl radical.

4. Indolizine derivatives according to any one of Claims 1 to 3, **characterized in that** R₃ and R₄ independently of each other represent a linear C₁-C₄ alkyl radical.

5. Indolizine derivatives according to any one of Claims 1 to 4, **characterized in that** R₃ and R₄ together form a piperidinyl or piperazinyl group optionally carrying one or more methyl radicals.

6. Indolizine derivatives according to any one of Claims 1 to 5, **characterized in that** R₂ represents a hydrogen atom.

7. Indolizine derivatives according to any one of Claims 1 to 6, **characterized in that** the pharmaceutically acceptable salt is selected from the oxalate and the hydrochloride.

8. Indolizine derivatives with formula (I) according to Claim 1, with the following names:
(2-butylindolizin-3-yl)(4-[3-(diethylamino)propyl]phenyl}methanone hydrochloride;
(2-ethylindolizin-3-yl){4-[3-(diethylamino)propyl]phenyl}methanone oxalate;
{4-[3-(diethylamino)propyl]phenyl}(2-propylindolizin-3-yl)methanone hydrochloride;
(2-butylindolizin-3-yl){4-[4-(diethylamino)butyl]phenyl}methanone oxalate;
{4-[4-(diethylamino)butyl]phenyl}(2-ethylindolizin-3-yl)methanone oxalate;
{4-[4-(diethylamino)butyl]phenyl}(2-propylindolizin-3-yl)methanone hydrochloride;
(2-butylindolizin-3-yl){4-[3-(dipropylamino)propyl]phenyl}methanone hydrochloride;
(2-butylindolizin-3-yl){4-[3-(dipropylamino)propyl]phenyl}methanone hydrochloride hemihydrate;
(2-butylindolizin-3-yl){4-[3-(dipropylamino)propyl]phenyl}methanone sulphate;
(2-butylindolizin-3-yl){4-[3-(dipropylamino)propyl]phenyl}methanone fumarate;
{4-[3-(dipropylamino)propyl]phenyl}(2-ethylindolizin-3-yl)methanone hydrochloride;
{4-[3-(dipropylamino)propyl]phenyl}(2-propylindolizin-3-yl)methanone hydrochloride;
(2-butylindolizin-3-yl){4-[4-(dipropylamino)butyl]phenyl}methanone hydrochloride;
{4-[4-(dipropylamino)butyl]phenyl}(2-ethylindolizin-3-yl)methanone hydrochloride;
4-[3-(dipropylamino)propyl]phenyl}(2-isopropylindolizin-3-yl)methanone hydrochloride;
{4-[3-(dipropylamino)propyl]phenyl}(2-methylindolizin-3-yl)methanone hydrochloride;
{4-[4-(dipropylamino)butyl]phenyl}(2-isopropylindolizin-3-yl)methanone oxalate;
{4-[4-(dipropylamino)butyl]phenyl}(2-methylindolizin-3-yl)methanone hydrochloride;
{4-[4-(dipropylamino)butyl]phenyl}(2-propylindolizin-3-yl)methanone hydrochloride;
(2-butylindolizin-3-yl){4-[3-(dibutylamino)propyl]phenyl}methanone hydrochloride;
{4-[3-(dibutylamino)propyl]phenyl}(2-ethylindolizin-3-yl)methanone hydrochloride;
{4-[3-(dibutylamino)propyl]phenyl}(2-propylindolizin-3-yl)methanone hydrochloride;
(2-butylindolizin-3-yl){4-[4-(dibutylamino)butyl]phenyl}methanone oxalate;
{4-[4-(dibutylamino)butyl]phenyl}(2-ethylindolizin-3-yl)methanone oxalate;
{4-[4-(dibutylamino)butyl]phenyl}(2-propylindolizin-3-yl)methanone oxalate;
(2-butylindolizin-3-yl)(4-{3-[ethyl(propyl)amino]propyl}phenyl)methanone hydrochloride;
(2-ethylindolizin-3-yl)(4-{3-[ethyl(propyl)amino]propyl}phenyl)methanone oxalate;
(4-{3-[ethyl(propyl)amino]propyl}phenyl)(2-isopropylindolizin-3-yl)methanone hydrochloride;
(4-{3-[ethyl(propyl)amino]propyl}phenyl)(2-methylindolizin-3-yl)methanone hydrochloride;
(4-{4-[ethyl(propyl)amino]butyl}phenyl)(2-isopropylindolizin-3-yl)methanone oxalate;
(4-{4-[ethyl(propyl)amino]butyl}phenyl)(2-methylindolizin-3-yl)methanone hydrochloride;
(4-{3-[ethyl(propyl)amino]propyl}phenyl)(2-propylindolizin-3-yl)methanone hydrochloride;
(2-butylindolizin-3-yl)(4-{4-[ethyl(propyl)amino]butyl}phenyl)methanone hydrochloride;
(2-ethylindolizin-3-yl)(4-{4-[ethyl(propyl)amino]butyl}phenyl)methanone oxalate;
(4-{4-[ethyl(propyl)amino]butyl}phenyl)(2-propylindolizin-3-yl)methanone hydrochloride;
(2-butylindolizin-3-yl)(4-{3-[butyl(propyl)amino]propyl}phenyl)methanone oxalate;
(4-{3-[butyl(ethyl)amino]propyl}phenyl)(2-butylindolizin-3-yl)methanone oxalate;
(2-butylindolizin-3-yl)(4-{3-[(cyclopropylmethyl)(propyl)amino]propyl}phenyl)me thanone hydrochloride;
{4-[4-(propylamino)butyl]phenyl}(2-propylindolizin-3-yl)methanone hydrochloride;
(2-butylindolizin-3-yl){4-[3-(propylamino)propyl]phenyl}methanone hydrochloride;
(2-butylindolizin-3-yl){4-[3-(dimethylamino)propyl]phenyl}methanone hydrochloride;
(2-butylindolizin-3-yl)(4-{3-[(3*R*,5*S*)-3,5-dimethylpiperazin-1-yl]propyl}phenyl)methanone hydrochloride;
(2-butylindolizin-3-yl)(4-{3-[(3*R*,5*S*)-3,4,5-trimethylpiperazin-1-yl]propyl}phenyl)methanone hydrochloride;
(4-{3-[bis(2-methoxyethyl)amino]propyl}phenyl)(2-butylindolizin-3-yl)methanone oxalate;
(2-butylindolizin-3-yl)[4-(3-piperidin-1-ylpropyl)phenyl]methanone oxalate;
(2-butyl-6-methylindolizin-3-yl){4-[3-(dipropylamino)propyl]phenyl}methanone hydrochloride;
(2-butyl-6-ethylindolizin-3-yl){4-[3-(dipropylamino)propyl]phenyl}methanone hydrochloride;
(2-butyl-6-methylindolizin-3-yl)(4-{3-[ethyl(propyl)amino]propyl}phenyl)methanone hydrochloride;
(2-butyl-6-ethylindolizin-3-yl)(4-{3-[ethyl(propyl)amino]propyl}phenyl)methanone hydrochloride;

9. Method for preparing derivatives with formula (I) as defined in any one of Claims 1 to 4, comprising the reaction of an amine H-Am with a compound represented by formula (II): 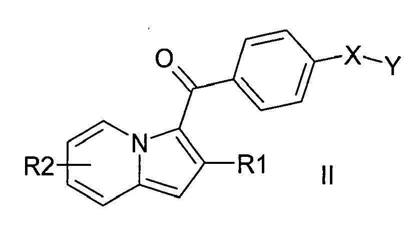 in which Y represents a halogen atom and the other substituents are as defined in the preceding claims.

10. Method comprising a method for preparing a compound with formula (II) according to Claim 9 and comprising the reaction of a compound represented by formula (III): 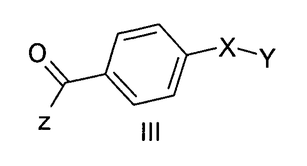 with a compound represented by formula (IV):

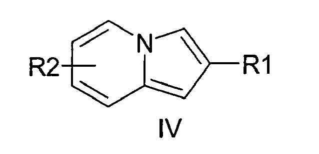

11. Indolizine derivatives with general formula (I) as defined in any one of Claims 1 to 8, for use as a drug.

12. Drugs comprising indolizine derivatives with general formula (I) as defined in any one of Claims 1 to 8.

13. Pharmaceutical or veterinary composition, **characterized in that** it contains, as the active principle, at least one indolizine derivative with formula (I) according to any one of Claims 1 to 8, or a pharmaceutically acceptable salt thereof, in association with a pharmaceutical vehicle or an appropriate excipient.

14. Use of an indolizine derivative with general formula (I) as defined in any one of Claims 1 to 8, to prepare a drug intended for the treatment of pathological syndromes of the cardiovascular system, in particular in the treatment of angina pectoris, hypertension, ventricular or supraventricular arrhythmia, the treatment of cardiac insufficiency, myocardial infarction complicated or otherwise by cardiac insufficiency or for the prevention of post-infarction mortality.

15. Use of an indolizine derivative according to Claim 14 to prepare a drug for the treatment of angina pectoris, hypertension, ventricular or supraventricular arrhythmia, the treatment of cardiac insufficiency, myocardial infarction complicated or otherwise by cardiac insufficiency or for the prevention of post-infarction mortality.

## Patentansprüche

1. Indolizinderivate der allgemeinen Formel (I) 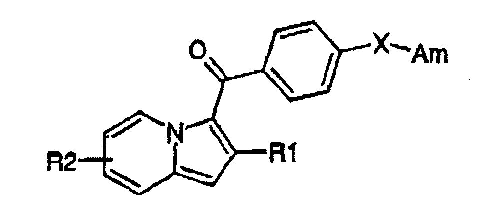 worin
X für einen -(CH₂)ₙ-Rest, wobei n eine ganze Zahl von 1 bis 6 ist, steht,
R₁ für einen linearen, verzweigten oder cyclischen C₁-C₈-Alkylrest steht,
Am für eine NR₃R₄-Gruppe steht, in der R₃ und R₄ gleich oder verschieden sind und unabhängig voneinander für
- ein Wasserstoffatom,
- einen linearen, verzweigten oder cyclischen C₁-C₆-Alkylrest oder einen durch einen C₃-C₆-Cycloalkylrest substituierten C₁-C₃-Alkylrest,
- oder einen (CH₂)₁-O-B-Rest, worin B für ein Wasserstoffatom oder einen (CH₂)ₖ-Alkylrest steht, wobei 1 und k für ganze Zahlen 1 ≥ 2 und 1+k ≤ 6 sind,
stehen oder R₃ und R₄ gemeinsam mit dem Stickstoffatom, an das sie gebunden sind, einen 4- bis 6-gliedrigen Heterocyclus bilden, der gegebenenfalls ein oder mehrere unter N und O ausgewählte Heteroatome enthalten kann und gegebenenfalls einen oder mehrere unter einem linearen, verzweigten oder cyclischen C₁-C₆-Alkylrest ausgewählte Substituenten tragen kann,
R₂ für ein Wasserstoffatom oder einen linearen, verzweigten oder cyclischen C₁-C₆-Alkylrest steht,
und pharmazeutisch akzeptable Salze davon.

2. Indolizinderivate nach Anspruch 1, **dadurch gekennzeichnet, daß** X für einen (CH₂)ₙ-Rest, wobei n eine ganze Zahl von 3 bis 4 ist, steht.

3. Indolizinderivate nach Anspruch 1 oder 2, **dadurch gekennzeichnet, daß** R₁ für einen C₁-C₄-Alkylrest steht.

4. Indolizinderivate nach einem der Ansprüche 1 bis 3, **dadurch gekennzeichnet, daß** R₃ und R₄ unabhängig voneinander für einen linearen C₁-C₄-Alkylrest stehen.

5. Indolizinderivate nach einem der Ansprüche 1 bis 4, **dadurch gekennzeichnet, daß** R₃ und R₄ gemeinsam eine Piperidinyl- oder Piperazinylgruppe, die gegebenenfalls einen oder mehrere Methylreste trägt, bilden.

6. Indolizinderivate nach einem der Ansprüche 1 bis 5, **dadurch gekennzeichnet, daß** R₂ für ein Wasserstoffatom steht.

7. Indolizinderivate nach einem der Ansprüche 1 bis 6, **dadurch gekennzeichnet, daß** das pharmazeutisch akzeptable Salz unter Oxalat und Hydrochlorid ausgewählt ist.

8. Indolizinderivate der Formel (I) nach Anspruch 1 mit den folgenden Namen:
(2-Butylindolizin-3-yl){4-[3-(diethylamino)propyl]phenyl}methanon-hydrochlorid
(2-Ethylindolizin-3-yl){4-[3-(diethylamino)propyl]phenyl}methanon-oxalat
{4-[3-(Diethylamino)propyl]phenyl}(2-propylindolizin-3-yl)methanon-hydrochlorid
(2-Butylindolizin-3-yl){4-[4-(diethylamino)butyl]phenyl}methanon-oxalat
{4-[4-(Diethylamino)butyl]phenyl}(2-ethylindolizin-3-yl)methanon-oxalat
{4-[4-(Diethylamino)butyl]phenyl}(2-propylindolizin-3-yl)methanon-hydrochlorid
(2-Butylindolizin-3-yl){4-[3-(dipropylamino)propyl]phenyl}methanon-hydrochlorid
(2-Butylindolizin-3-yl){4-[3-(dipropylamino)propyl]phenyl}methanon-hydrochlorid-hemihydrat
(2-Butylindolizin-3-yl){4-[3-(dipropylamino)propyl]phenyl}methanon-sulfat
(2-Butylindolizin-3-yl){4-[3-(dipropylamino)propyl]phenyl}methanon-fumarat
{4-[3-(Dipropylamino)propyl]phenyl}(2-ethylindolizin-3-yl)methanon-hydrochlorid
{4-[3-(Dipropylamino)propyl]phenyl}(2-propylindolizin-3-yl)methanon-hydrochlorid
(2-Butylindolizin-3-yl){4-[4-(dipropylamino)butyl]phenyl}methanon-hydrochlorid
{4-[4-(Dipropylamino)butyl]phenyl}(2-ethylindolizin-3-yl)methanon-hydrochlorid
4-[3-(Dipropylamino)propyl]phenyl}(2-isopropylindolizin-3-yl)methanon-hydrochlorid
{4-[3-(Dipropylamino)propyl]phenyl}(2-methylindolizin-3-yl)methanon-hydrochlorid
{4-[4-(Dipropylamino)butyl]phenyl}(2-isopropylindolizin-3-yl)methanon-oxalat
{4-[4-(Dipropylamino)butyl]phenyl}(2-methylindolizin-3-yl)methanon-hydrochlorid
{4-[4-(Dipropylamino)butyl]phenyl}(2-propylindolizin-3-yl)methanon-hydrochlorid
(2-Butylindolizin-3-yl){4-[3-(dibutylamino)propyl]phenyl}methanon-hydrochlorid
{4-[3-(Dibutylamino)propyl]phenyl}(2-ethylindolizin-3-yl)methanon-hydrochlorid
{4-[3-(Dibutylamino)propyl]phenyl}(2-propylindolizin-3-yl)methanon-hydrochlorid
(2-Butylindolizin-3-yl){4-[4-(dibutylamino)butyl]phenyl}methanon-oxalat
{4-[4-(Dibutylamino)butyl]phenyl}(2-ethylindolizin-3-yl)methanon-oxalat
{4-[4-(Dibutylamino)butyl]phenyl}(2-propylindolizin-3-yl)methanon-oxalat
(2-Butylindolizin-3-yl)(4-{3-[ethyl(propyl)amino]propyl}phenyl)methanon-hydrochlorid
(2-Ethylindolizin-3-yl)(4-{3-[ethyl(propyl)amino]propyl}phenyl)methanon-oxalat
(4-{3-[Ethyl(propyl)amino]propyl}phenyl)(2-isopropylindolizin-3-yl)methanon-hydrochlorid
(4-{3-[Ethyl(propyl)amino]propyl}phenyl)(2-methylindolizin-3-yl)methanon-hydrochlorid
(4-{4-[Ethyl(propyl)amino]butyl}phenyl)(2-isopropylindolizin-3-yl)methanon-oxalat
(4-{4-[Ethyl(propyl)amino]butyl}phenyl)(2-methylindolizin-3-yl)methanon-hydrochlorid
(4-{3-[Ethyl(propyl)amino]propyl}phenyl)(2-propylindolizin-3-yl)methanon-hydrochlorid
(2-Butylindolizin-3-yl)(4-{4-[ethyl(propyl)amino]butyl}phenyl)methanon-hydrochlorid
(2-Ethylindolizin-3-yl)(4-{4-[ethyl(propyl)amino]butyl}phenyl)methanon-oxalat
(4-{4-[Ethyl(propyl)amino]butyl}phenyl)(2-propylindolizin-3-yl)methanon-hydrochlorid
(2-Butylindolizin-3-yl)(4-{3-[butyl(propyl)amino]propyl}phenyl)methanon-oxalat
(4-{3-[Butyl(ethyl)amino]propyl}phenyl)(2-butylindolizin-3-yl)methanon-oxalat
(2-Butylindolizin-3-yl)(4-{3-[(cyclopropylmethyl)(propyl)amino]propyl}phenyl)methanon-hydrochlorid
{4-[4-(Propylamino)butyl]phenyl}(2-propylindolizin-3-yl)methanon-hydrochlorid
(2-Butylindolizin-3-yl){4-[3-(propylamino)propyl]phenyl}methanon-hydrochlorid
(2-Butylindolizin-3-yl){4-[3-(dimethylamino)propyl]phenyl}methanon-hydrochlorid
(2-Butylindolizin-3-yl)(4-{3-[(3*R*,5*S*)-3,5-dimethylpiperazin-1-yl]propyl}phenyl)methanon-hydrochlorid
(2-Butylindolizin-3-yl)(4-{3-[(3*R*,5*S*)-3,4,5-trimethylpiperazin-1-yl]propyl}phenyl)methanon-hydrochlorid
(4-{3-[Bis(2-methoxyethyl)amino]propyl}phenyl)(2-butylindolizin-3-yl)methanon-oxalat
(2-Butylindolizin-3-yl)[4-(3-piperidin-1-yl-propyl)phenyl]methanon-oxalat
(2-Butyl-6-methylindolizin-3-yl){4-[3-(dipropyl-amino)propyl]phenyl}methanon-hydrochlorid
(2-Butyl-6-ethylindolizin-3-yl){4-[3-(dipropyl-amino)propyl]phenyl}methanon-hydrochlorid
(2-Butyl-6-methylindolizin-3-yl)(4-{3-[ethyl(propyl)amino]propyl}phenyl)methanon-hydrochlorid
(2-Butyl-6-ethylindolizin-3-yl)(4-{3-[ethyl(propyl)amino]propyl}phenyl)methanon-hydrochlorid.

9. Verfahren zur Herstellung der Derivate der Formel (I) gemäß einem der Ansprüche 1 bis 4, bei dem man ein Amin H-Am mit einer Verbindung der Formel (II) : 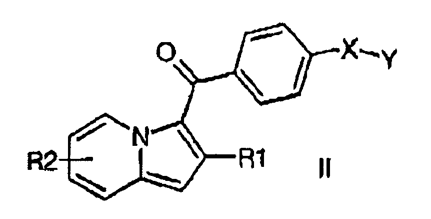 worin Y für ein Halogenatom steht und die anderen Substituenten die in den vorhergehenden Ansprüchen angegebene Bedeutung besitzen, umsetzt.

10. Verfahren, umfassend ein Verfahren zur Herstellung der Verbindung der Formel (II) nach Anspruch 9, bei dem man eine Verbindung der Formel (III): 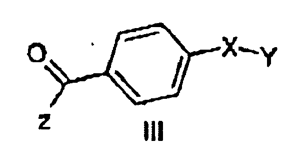 mit einer Verbindung der Formel (IV): 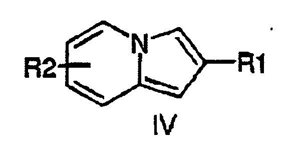 umsetzt.

11. Indolizinderivate der allgemeinen Formel (I) gemäß einem der Ansprüche 1 bis 8 zur Verwendung als Arzneimittel.

12. Arzneimittel, enthaltend die Indolizinderivate der allgemeinen Formel (I) gemäß einem der Ansprüche 1 bis 8.

13. Pharmazeutische oder veterinärmedizinische Zusammensetzung, **dadurch gekennzeichnet, daß** sie als Wirkstoff mindestens ein Indolizinderivat der Formel (I) nach einem der Ansprüche 1 bis 8 oder ein pharmazeutisch akzeptables Salz davon in Kombination mit einem pharmazeutischen Träger oder einem geeigneten Trägerstoff enthält.

14. Verwendung eines Indolizinderivats der allgemeinen Formel (I) gemäß einem der Ansprüche 1 bis 8 zur Herstellung eines Arzneimittels zur Behandlung von pathologischen Syndromen des Herz-KreislaufSystems, insbesondere bei der Behandlung von Angina pectoris, Hypertonie, atrialer, ventrikulärer oder supraventrikulärer Arrhythmie, Behandlung von Herzinsuffizienz und Myokardinfarkt mit oder ohne Komplikation durch Herzinsuffizienz oder zur Prävention von Postinfarkt-Mortalität.

15. Verwendung eines Indolizinderivats nach Anspruch 14 zur Herstellung eines Arzneimittels zur Behandlung von Angina pectoris, Hypertonie, atrialer, ventrikulärer oder supraventrikulärer Arrhythmie, Behandlung von Herzinsuffizienz und Myokardinfarkt mit oder ohne Komplikation durch Herzinsuffizienz oder zur Prävention von Postinfarkt-Mortalität.
